# EUROPEAN PATENT APPLICATION

(11) **EP 4 800 077 A1**
(43) Date of publication of application: **02.09.2026**
(21) Application number: 24882138.1
(22) Date of filing: 07.10.2024
(51) Int. Cl.: C09B 11/28, B41M 5/327, C07D 519/00

(54) **FLUORAN DIMER-BASED COMPOUND AND RECORDING MATERIAL USING SAME**

(30) Priority: 27.10.2023 JP 2023184457
(71) Applicant: Yamamoto Chemicals, Inc., Yao-shi, Osaka 581-0034 (JP)
(72) Inventor: KUMAGAE, Yojiro, Yao-shi, Osaka 581-0034 (JP); SASAKI, Hiroyuki, Yao-shi, Osaka 581-0034 (JP); NARUSE, Hiroshi, Yao-shi, Osaka 581-0034 (JP)
(74) Representative: Hoffmann Eitle
(86) International application number: PCT/JP2024/035770
(87) International publication number: WO 2025/089021

(57) **Abstract**

The fluoran dimer compound according to the present invention is represented by General Formula (1).

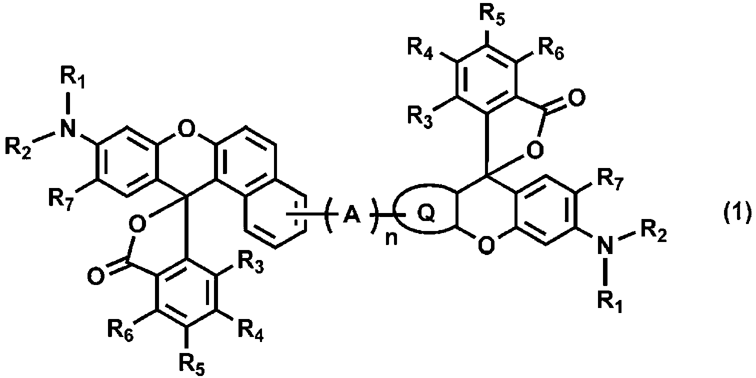

## Description

### TECHNICAL FIELD

The present invention relates to a fluoran dimer compound and a recording material containing the same. More specifically, the present invention relates to a fluoran dimer compound suitable for a recording material capable of recording an image or data in a magenta color (crimson) by reacting with an electron-accepting color developer through use of a photothermal conversion material that generates heat by near-infrared light of a thermal head or a laser.

### BACKGROUND ART

In recent years, the development of rewritable recording material technology has been remarkable from the viewpoint of earth environment preservation. As a reversible recording medium capable of reversibly recording or erasing information by heat or light, various prepaid cards, point cards, credit cards, IC cards, and the like have been widely used, and the use of a reversible recording medium has been studied even in the field of displays.

At the same time, full colorization has been progressing from the viewpoint of designability. In particular, a color forming agent that develops a clear magenta color (crimson) among the primary colors and has excellent durability before and after color development is required.

For example, Patent Document 1 and Patent Document 2 disclose a fluoran compound that develops a magenta color (crimson).

However, in order to be used as a color forming agent of a full-color thermosensitive recording material, the light fastness before and after color development is low, and the color change occurs, so that improvement is required.

On the other hand, Patent Document 3, Patent Document 4, Patent Document 5, and Non-Patent Document 1 disclose a fluoran dimer compound. However, since the color development hue is not a clear magenta color (crimson) in any of them, improvement is required.

### RELATED DOCUMENT

### PATENT DOCUMENT

Patent Document 1: Japanese Unexamined Patent Publication No. S50-5117
Patent Document 2: Japanese Unexamined Patent Publication No. H10-109477
Patent Document 3: US4012419A
Patent Document 4: US5618063A
Patent Document 5: Japanese Unexamined Patent Publication No. H11-245523

### NON-PATENT DOCUMENT

Non-Patent Document 1: Journal of the Serbian Chemical Society (2003), 68(8-9), 607-613

### SUMMARY OF THE INVENTION

### TECHNICAL PROBLEM

An object of the present invention is to provide a fluoran dimer compound which develops a vivid magenta color (crimson) and has high light fastness and the like before and after color development and suppressed deterioration, and further to provide a recording material containing the compound.

### SOLUTION TO PROBLEM

As a result of studies on the above-described object, the present inventors have found that the object can be achieved by incorporating a fluoran dimer compound having a specific structure into a color development composition, thereby completing the present invention.

That is, the present invention is as follows.
[1] A fluoran dimer compound represented by General Formula (1), 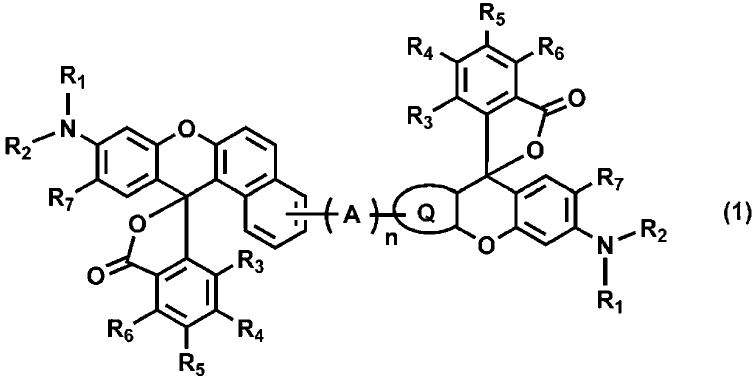
   in which, in Formula (1), R₁ and R₂ each independently represent a hydrogen atom, a substituted or unsubstituted alkyl group, a substituted or unsubstituted aralkyl group, or a substituted or unsubstituted aryl group, and R₁ and R₂ are optionally bonded to each other to form an aliphatic ring; R₃ to R₆ each independently represent a hydrogen atom, a halogen atom, a substituted or unsubstituted alkyl group, a substituted or unsubstituted alkoxy group, a substituted or unsubstituted aryloxy group, a substituted or unsubstituted alkylthio group, or a substituted or unsubstituted arylthio group, and R₃ to R₆ are optionally bonded to each other to form an aliphatic ring or an aromatic ring;
   R₇ represents a hydrogen atom or an unsubstituted alkyl group;
   Q represents an unsubstituted benzene ring or an unsubstituted naphthalene ring; A is selected from a substituted or unsubstituted arylene group or a group represented by General Formula (2); and n represents 0 or 1, 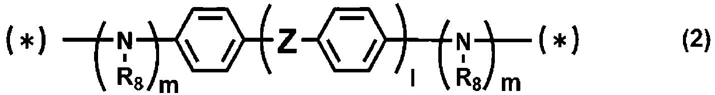
   in which, in Formula (2), Z represents an oxygen atom, a sulfur atom, a phenylene group, a ketone group, or a substituted or unsubstituted alkylene group, R₈ represents a hydrogen atom or an unsubstituted alkyl group; l and m each independently represent 0 or 1, in a case where l is 0, m represents 1, and in a case where m is 0, l represents 1; and (*) represents a bonding position.
[2] The fluoran dimer compound according to [1], in which Q is an unsubstituted benzene ring.
[3] The fluoran dimer compound according to [1], in which Q is an unsubstituted naphthalene ring.
[4] The fluoran dimer compound according to any one of [1] to [3], in which R₁ and R₂ each independently represent a hydrogen atom, a substituted or unsubstituted alkyl group having 1 to 20 carbon atoms, a substituted or unsubstituted aralkyl group having 7 to 25 carbon atoms, or a substituted or unsubstituted aryl group having 6 to 24 carbon atoms, and R₁ and R₂ are optionally bonded to each other to form a substituted or unsubstituted heterocyclic aliphatic ring having 4 to 20 carbon atoms.
[5] The fluoran dimer compound according to any one [1] to [4], in which R₃ to R₆ each independently represent a hydrogen atom, a halogen atom, a substituted or unsubstituted alkyl group having 1 to 20 carbon atoms, a substituted or unsubstituted alkoxy group having 1 to 25 carbon atoms, a substituted or unsubstituted aryloxy group having 6 to 25 carbon atoms, a substituted or unsubstituted alkylthio group having 1 to 25 carbon atoms, or a substituted or unsubstituted arylthio group having 6 to 25 carbon atoms, and R₃ to R₆ are optionally bonded to each other to form a substituted or unsubstituted carbocyclic aliphatic ring having 5 to 20 carbon atoms or a substituted or unsubstituted carbocyclic aromatic ring having 6 to 20 carbon atoms.
[6] The fluoran dimer compound according to any one of [1] to [5], in which R₇ represents a hydrogen atom or an unsubstituted alkyl group having 1 to 8 carbon atoms.
[7] The fluoran dimer compound according to any one of [1] to [6],
   in which A is a substituted or unsubstituted arylene group having 6 to 18 carbon atoms or a group represented by General Formula (2),
   in which, in General Formula (2), Z represents an oxygen atom, a sulfur atom, a ketone group, or a substituted or unsubstituted alkylene group having 1 to 20 carbon atoms, and R₈ represents a hydrogen atom or an alkyl group having 1 to 4 carbon atoms.
[8] A recording material including:
   an electron-donating color forming agent; and an electron-accepting color developer, in which a color-forming reaction between the electron-donating color forming agent and the electron-accepting color developer is utilized, and the electron-donating color forming agent includes at least one of the fluoran dimer compounds according to any one of [1] to [7].
[9] A thermosensitive recording material comprising the recording material according to [8].

### ADVANTAGEOUS EFFECTS OF INVENTION

The fluoran dimer compound according to the present invention is colored in a vivid magenta color (crimson) by a reaction (interaction) with a color developer. Therefore, in a case where the fluoran dimer compound according to the present invention is used as a recording material, a colorless or substantially colorless image before color development and a vivid magenta color developed color image after color development can be provided. Further, since the recording material has excellent storage stability before color development and has high light fastness of the image after color development, a vivid magenta color developed color image can be stably provided. As described above, the fluoran dimer compound according to the present invention can be suitably used as a magenta color (crimson) color-forming material of a full-color thermosensitive recording material.

### DESCRIPTION OF EMBODIMENTS

Hereinafter, the present invention will be described in detail.

The fluoran dimer compound according to the present invention is contained as at least one electron-donating color forming agent in a recording material using a color-forming reaction between an electron-donating color forming agent and an electron-accepting color developer.

First, the fluoran dimer compound will be described below.

### [Fluoran dimer compound]

The fluoran dimer compound according to the present invention is represented by General Formula (1).

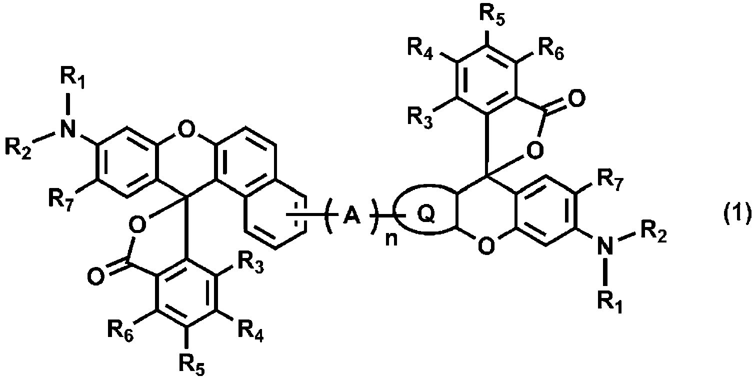

In Formula (1), R₁ and R₂ each independently represent a hydrogen atom, a substituted or unsubstituted alkyl group, a substituted or unsubstituted aralkyl group, or a substituted or unsubstituted aryl group. R₁ and R₂ are optionally bonded to each other to form an aliphatic ring.

R₃ to R₆ each independently represent a hydrogen atom, a halogen atom, a substituted or unsubstituted alkyl group, a substituted or unsubstituted alkoxy group, a substituted or unsubstituted aryloxy group, a substituted or unsubstituted alkylthio group, or a substituted or unsubstituted arylthio group. R₃ to R₆ are optionally bonded to each other to form an aliphatic ring or an aromatic ring.

R₇ represents a hydrogen atom or an unsubstituted alkyl group.

Q represents an unsubstituted benzene ring or an unsubstituted naphthalene ring.

A is selected from a substituted or unsubstituted arylene group or a group represented by General Formula (2), and n represents 0 or 1.

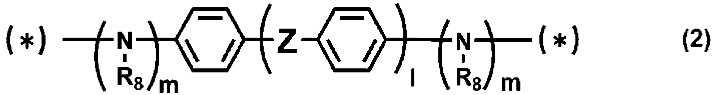

In Formula (2), Z represents an oxygen atom, a sulfur atom, a phenylene group, a ketone group, or an alkylene group, R₈ represents a hydrogen atom or an unsubstituted alkyl group, and l and m each independently represent 0 or 1. However, in a case where l represents 0, m represents 1, and in a case where m represents 0, l represents 1. (*) represents a bonding position.

In R₁ and R₂ represented by Formula (1),
examples of the substituted or unsubstituted alkyl group include an alkyl group having 1 to 20 carbon atoms, preferably 1 to 12 carbon atoms, and more preferably 1 to 8 carbon atoms.

Examples of the unsubstituted alkyl group include linear, branched, or cyclic unsubstituted alkyl groups such as a methyl group, an ethyl group, an n-propyl group, an isopropyl group, an n-butyl group, an isobutyl group, a sec-butyl group, a tert-butyl group, an n-pentyl group, an isopentyl group, a neopentyl group, a sec-pentyl group, a tert-pentyl group, an n-hexyl group, a 1-methylpentyl group, a 4-methylpentyl group, a 2-ethylbutyl group, an n-heptyl group, a 1-methylhexyl group, an n-octyl group, a tert-octyl group, a 1-methylheptyl group, a 2-ethylhexyl group, a cyclopentyl group, a cyclohexyl group, a 4-methylcyclohexyl group, a 3-methylcyclohexyl group, a 2-methylcyclohexyl group, a 4-ethylcyclohexyl group, a 4-isopropylcyclohexyl group, a 3,3,5-trimethylcyclohexyl group, a 4-butylcyclohexyl group, a 4-tert-butylcyclohexyl group, a 4-sec-butylcyclohexyl group, and a 4-amylcyclohexyl group.

Examples of the substituted alkyl group include alkyl groups having a linear or cyclic alkyloxy group, such as a methoxymethyl group, an ethoxymethyl group, an ethoxyethyl group, a (2-methoxyethoxy)ethyl group, an n-butoxymethyl group, an n-hexyloxymethyl group, a (2-ethylbutoxy)methyl group, a 3-methoxypropyl group, and a tetrahydrofuranyl methyl group;
alkyl groups having an alkenyloxy group, such as a 2-(4'-pentenyloxy)ethyl group;
alkyl groups having an alkylamino group, such as a 4-dimethylaminocyclohexyl group;
alkyl groups having an aralkyloxy group, such as a benzyloxymethyl group and a 2-(benzyloxymethoxy)ethyl group;
alkyl groups having an aryloxy group, such as a phenyloxymethyl group, a 4-chlorophenyloxymethyl group, and a 4-(2'-phenyloxyethoxy)butyl group;
alkyl groups having an alkylthio group, such as an n-butylthiomethyl group and a 2-n-octylthioethyl group;
alkyl groups having a halogen atom, such as a fluoromethyl group, a trifluoromethyl group, a perfluoroethyl group, a 2,2,3,3,3-pentafluoropropyl group, a 2,2,4,4,4-pentafluorobutyl group, a 4-fluorocyclohexyl group, a dichloromethyl group, a 4-chlorocyclohexyl group, a 7-chloroheptyl group, a 4-trifluoromethylcyclohexyl group, and a 3-trifluoromethylcyclohexyl group.

Examples of the substituted or unsubstituted aralkyl group include aralkyl groups having 7 to 25 carbon atoms, preferably 7 to 20 carbon atoms, and specifically include unsubstituted aralkyl groups, or aralkyl groups having an alkyl group, such as a benzyl group, an α-methylbenzyl group, a phenethyl group, an α-methylphenethyl group, an α,α-dimethylbenzyl group, an α,α-dimethylphenethyl group, a 4-methylphenethyl group, a 4-methylbenzyl group, and a 4-isopropylbenzyl group;
aralkyl groups having an aryl group or an aralkyl group, such as a 4-benzylbenzyl group, a 4-phenethylbenzyl group, and a 4-phenylbenzyl group;
aralkyl groups having a substituted oxy group, such as a 4-methoxybenzyl group, a 4-n-tetradecyloxybenzyl group, a 4-n-heptadecyloxybenzyl group, a 3,4-dimethoxybenzyl group, a 4-methoxymethylbenzyl group, a 4-vinyloxy methylbenzyl group, a 4-benzyloxybenzyl group, and a 4-phenethyloxybenzyl group;
aralkyl groups having a halogen atom, such as a 4-fluorobenzyl group, a 3-chlorobenzyl group, and a 3,4-dichlorobenzyl group;
a 2-furfuryl group, a diphenylmethyl group, a 1-naphthylmethyl group, and a 2-naphthylmethyl group.

Examples of the substituted or unsubstituted aryl group include aryl groups having 6 to 24 carbon atoms, preferably 6 to 19 carbon atoms. In the present specification, the aryl group represents, for example, a carbocyclic aromatic group such as a phenyl group and a naphthyl group, and a heterocyclic aromatic group such as a furyl group, a thienyl group, a pyridyl group, and a pyrazyl group.

Examples of the unsubstituted aryl group include a phenyl group, a 1-naphthyl group, a 2-naphthyl group, a 2-anthracenyl group, a 1-phenanthryl group, a 2-phenanthryl group, a 3-phenanthryl group, a 1-pyrenyl group, a 2-pyrenyl group, a 2-perylenyl group, a 3-perylenyl group, a 2-fluoranthenyl group, a 3-fluoranthenyl group, a 7-fluoranthenyl group, a 8-fluoranthenyl group, a 2-furyl group, a 2-thienyl group, a 3-thienyl group, a 2-pyridyl group, a 3-pyridyl group, a 4-pyridyl group, a 2-methylpyrazyl group, a 4-methylpyrazyl group, and a 5-methylpyrazyl group.

Examples of the substituted aryl group include aryl groups having an alkyl group, such as a 1-methyl-2-pyrenyl group, a 2-methylphenyl group, a 4-methylphenyl group, a 4-ethylphenyl group, a 4-tert-butylphenyl group, a 4-(4'-tert-butylcyclohexyl)phenyl group, a 3-cyclohexylphenyl group, a 2-cyclohexylphenyl group, a 4-ethyl-1-naphthyl group, a 6-n-butyl-2-naphthyl group, a 2,4-dimethylphenyl group, a 5-ethyl-2-thienyl group, a 5-n-pentyl-2-thienyl group, a 5-n-decyl-2-thienyl group, a 2-methylpyrazyl group, a 4-methylpyrazyl group, and a 5-methylpyrazyl group;
aryl groups having an alkoxy group or an aryloxy group, such as a 4-methoxyphenyl group, a 3-ethoxyphenyl group, a 2-ethoxyphenyl group, a 4-n-propoxyphenyl group, a 3-n-propoxyphenyl group, a 4-isopropoxyphenyl group, a 3-isopropoxyphenyl group, a 2-isopropoxyphenyl group, a 2-sec-butoxyphenyl group, a 4-n-pentyloxyphenyl group, a 4-isopentyloxyphenyl group, a 2-methyl-5-methoxyphenyl group, and a 2-phenyloxyphenyl group;
aryl groups having an aryl group, such as a 4-phenylphenyl group, a 3-phenylphenyl group, a 2-phenylphenyl group, a 2,6-diphenylphenyl group, a 4-(2'-naphthyl)phenyl group, a 2-phenyl-1-naphthyl group, a 1-phenyl-2-naphthyl group, a 7-phenyl-1-pyrenyl group, a 5-phenyl-2-thienyl group, and a 5-(2'-thienyl)-2-thienyl group;
aryl groups having a halogen atom, such as a 4-fluorophenyl group, a 3-fluorophenyl group, a 2-fluorophenyl group, a 4-chlorophenyl group, a 4-bromophenyl group, a 2,4,6-trichlorophenyl group, a 2-chloro-5-methylphenyl group, a 2-chloro-6-methylphenyl group, a 2-methyl-3-chlorophenyl group, a 2-methoxy-4-fluorophenyl group, a 2-fluoro-4-methoxyphenyl group, a 2-fluoropyrazyl group, a 4-fluoropyrazyl group, and a 5-fluoropyrazyl group;
a 2-trifluoromethylphenyl group, a 3-trifluoromethylphenyl group, a 4-trifluoromethylphenyl group, a 3,5-bistrifluoromethylphenyl group, a 4-per-fluoroethylphenyl group, a 4-methylthiophenyl group, a 4-ethylthiophenyl group, a 4-cyanophenyl group, and a 3-cyanophenyl group.

R₁ and R₂ are optionally linked to each other to form a ring. In a case of forming a ring, the ring is usually formed to include a nitrogen atom to be bonded. Preferred examples of the ring formed in this way include a substituted or unsubstituted heterocyclic aliphatic ring, and more preferred examples thereof include a substituted or unsubstituted heterocyclic aliphatic ring having a total number of carbon atoms of 4 to 20.

Specific examples of the heterocyclic aliphatic ring include a pyrrolidine ring, a piperazine ring, a piperidine ring, a morpholine ring, and a thiomorpholine ring. The heterocyclic aliphatic ring to be formed may have a plurality of substituents.

Examples of the substituent include a halogen atom, a carboxyl group, a cyano group, a nitro group, a hydroxyl group, a linear, branched, or cyclic alkyl group having 1 to 20 carbon atoms, a linear, branched, or cyclic alkoxy group having 1 to 20 carbon atoms, and an aryl group which are optionally substituted with a halogen atom, a carboxyl group, a cyano group, a nitro group, a hydroxyl group, a linear, branched, or cyclic alkyl group having 1 to 20 carbon atoms, or a linear, branched, or cyclic alkoxy group having 1 to 20 carbon atoms.

From the viewpoint of the effect of the present invention, it is preferable that R₁ and R₂ in Formula (1) are each independently a hydrogen atom, a substituted or unsubstituted alkyl group having 1 to 20 carbon atoms, a substituted or unsubstituted aralkyl group having 7 to 25 carbon atoms, or a substituted or unsubstituted aryl group having 6 to 24 carbon atoms, or R₁ and R₂ are bonded to each other to form a substituted or unsubstituted heterocyclic aliphatic ring having 4 to 20 carbon atoms, and it is more preferable that R₁ and R₂ are a hydrogen atom or a substituted or unsubstituted alkyl group having 1 to 20 carbon atoms.

In R₃ to R₆ in Formula (1),
examples of the halogen atom include a chlorine atom, a fluorine atom, a bromine atom, and an iodine atom, and preferred examples thereof include a chlorine atom and a fluorine atom.

Examples of the substituted or unsubstituted alkyl group include the specific examples described above for R₁ and R₂, and preferred examples thereof are also the same.

Examples of the substituted or unsubstituted alkoxy group include an alkoxy group having 1 to 25 carbon atoms, preferably 1 to 15 carbon atoms.

Examples of the unsubstituted alkoxy group include a linear, branched, or cyclic unsubstituted alkoxy group such as a methoxy group, an ethoxy group, an n-propyloxy group, an isopropyloxy group, an n-butyloxy group, an isobutyloxy group, a tert-butyloxy group, a sec-butyloxy group, an n-pentyloxy group, an isopentyloxy group, an n-hexyloxy group, a 2-methylpentyloxy group, a 1,1-dimethylbutyloxy group, a 1,2,2-trimethylpropyloxy group, a 2-ethylbutyloxy group, a 1,3-dimethylhexyloxy group, a cyclohexyloxy group, a methylcyclopentyloxy group, an n-heptyloxy group, an n-octyloxy group, a 3,5,5-trimethylhexyloxy group, an n-decyloxy group, an n-undecyloxy group, an n-dodecyloxy group, a 1-adamantyloxy group, and an n-pentadecyloxy group.

Examples of the substituted alkoxy group include an alkoxy group having an alkoxy group such as a methoxymethoxy group, an ethoxymethoxy group, an n-propyloxymethoxy group, an n-butyloxymethoxy group, an isobutyloxymethoxy group, a tert-butyloxymethoxy group, an n-pentyloxymethoxy group, a 2-methoxyethoxy group, a 2-ethoxyethoxy group, a 2-n-propyloxyethoxy group, a 2-isopropyloxyethoxy group, a 2-n-butyloxyethoxy group, a 2-isobutyloxyethoxy group, a 2-tert-butyloxyethoxy group, a 2-sec-butyloxyethoxy group, a 2-n-pentyloxyethoxy group, a 2-isopentyloxyethoxy group, a 2-tert-pentyloxyethoxy group, a 2-sec-pentyloxyethoxy group, a 2-cyclopentyloxyethoxy group, a 2-n-hexyloxyethoxy group, a 2-(4-ethylcyclohexyloxy)ethoxy group, a 2-n-nonyloxyethoxy group, a 2-(3,5,5-trimethylhexyloxy)ethoxy group, a 2-n-decyloxyethoxy group, a 2-n-dodecyloxyethoxy group, a 3-methoxypropyloxy group, a 3-ethoxypropyloxy group, a 3-(n-propyloxy)propyloxy group, a 2-isopentyloxypropyloxy group, a 2-methoxybutyloxy group, a 4-ethoxybutyloxy group, a 4-(n-propyloxy)butyloxy group, a 4-isopropyloxybutyloxy group, a 5-methoxypentyloxy group, a 5-ethoxymethoxy group, and a 6-n-propylhexyloxy group; and
an alkoxy group having an alkoxy alkoxy group such as a methoxymethoxymethoxy group, an ethoxymethoxymethoxy group, a propyloxymethoxymethoxy group, a butyloxymethoxymethoxy group, a methoxyethoxymethoxy group, an ethoxyethoxymethoxy group, a propyloxyethoxymethoxy group, a methoxymethoxyethoxy group, an ethoxymethoxyethoxy group, a propyloxymethoxyethoxy group, a butyloxymethoxyethoxy group, a propyloxyethoxyethoxy group, a butyloxyethoxyethoxy group, a propyloxybutyloxyethoxy group, a methoxymethoxypropyloxy group, an ethoxymethoxypropyloxy group, a butyloxymethoxypropyloxy group, a methoxymethoxybutyloxy group, an ethoxymethoxybutyloxy group, a butyloxymethoxybutyloxy group, an ethoxyethoxybutyloxy group, a (4-ethylcyclohexyloxy)ethoxyethoxy group, and a [4-(3,5,5-trimethylhexyloxy)butyloxy]ethoxy group.

Examples of the substituted or unsubstituted aryloxy group include an aryloxy group having 6 to 25 carbon atoms, preferably 6 to 18 carbon atoms, and specific examples thereof include a phenyloxy group, a 2-methylphenyloxy group, a 4-methylphenyloxy group, a 4-ethylphenyloxy group, a 4-isopropylphenyloxy group, a 4-isobutylphenyloxy group, a 4-n-pentylphenyloxy group, a 4-tert-pentylphenyloxy group, a 4-cyclohexylphenyloxy group, a 4-n-octylphenyloxy group, a 4-n-decylphenyloxy group, a 4-n-dodecylphenyloxy group, a 4-n-hexadecylphenyloxy group, a 2,3-dimethylphenyloxy group, a 2,5-dimethylphenyloxy group, a 3,4-dimethylphenyloxy group, a 3,4,5-trimethylphenyloxy group, a 5-indanyloxy group, a 1,2,3,4-tetrahydro-6-naphthyloxy group, a 3-methoxyphenyloxy group, a 3-ethoxyphenyloxy group, a 4-n-propyloxyphenyloxy group, a 4-n-butoxyphenyloxy group, a 4-n-pentyloxyphenyloxy group, a 4-cyclohexyloxyphenyloxy group, a 4-n-octyloxyphenyloxy group, a 4-n-decyloxyphenyloxy group, a 4-n-dodecyloxyphenyloxy group, a 4-n-hexadecyloxyphenyloxy group, a 2,3-dimethoxyphenyloxy group, a 2,5-dimethoxyphenyloxy group, a 3,5-dimethoxyphenyloxy group, a 2-methoxy-4-methylphenyloxy group, a 3-methoxy-4-methylphenyloxy group, a 3-methyl-4-methoxyphenyloxy group, a 2-fluorophenyloxy group, a 4-fluorophenyloxy group, a 3-chlorophenyloxy group, a 4-bromophenyloxy group, a 3-trifluoromethylphenyloxy group, a 3,5-difluorophenyloxy group, a 3,4-dichlorophenyloxy group, a 2-methyl-4-chlorophenyloxy group, a 3-chloro-4-methylphenyloxy group, a 3-methoxy-4-fluorophenyloxy group, a 3-fluoro-4-methoxyphenyloxy group, a 4-phenylphenyloxy group, a 3-phenylphenyloxy group, a 4-(4'-methylphenyl)phenyloxy group, a 4-(4'-methoxyphenyl)phenyloxy group, a 1-naphthyloxy group, a 4-methyl-1-naphthyloxy group, a 6-n-butyl-2-naphthyloxy group, a 7-ethoxy-2-naphthyloxy group, a 2-thienyloxy group, a 2-pyridyloxy group, a 4-pyridyloxy group, a 2-furyloxy group, a 5-ethyl-2-thienyloxy group, a 5-n-pentyl-2-thienyloxy group, a 5-n-decyl-2-thienyloxy group, a 5-phenyl-2-thienyloxy group, a 5-(2'-thienyl)-2-thienyloxy group, a 3-thienyloxy group, and a 3-pyridyloxy group.

Examples of the substituted or unsubstituted alkylthio group include an alkylthio group having 1 to 25 carbon atoms, preferably 1 to 15 carbon atoms.

Examples of the unsubstituted alkylthio group include a linear, branched, or cyclic unsubstituted alkylthio group such as a methylthio group, an ethylthio group, an n-propylthio group, an isopropylthio group, an n-butylthio group, an isobutylthio group, a tert-butylthio group, a sec-butylthio group, an n-pentylthio group, an isopentylthio group, an n-hexylthio group, a 2-methylpentylthio group, a 1,1-dimethylbutylthio group, a 1,2,2-trimethylpropylthio group, a 2-ethylbutylthio group, a 1,3-dimethylhexylthio group, a cyclohexylthio group, a methylcyclopentylthio group, an n-heptylthio group, an n-octylthio group, a 3,5,5-trimethylhexylthio group, an n-decylthio group, an n-undecylthio group, an n-dodecylthio group, a 1-adamantylthio group, and an n-pentadecylthio group.

Examples of the substituted alkylthio group include an alkylthio group having an alkoxy group such as a methoxymethylthio group, an ethoxymethylthio group, an n-propyloxymethylthio group, an n-butyloxymethylthio group, an isobutyloxymethylthio group, a tert-butyloxymethylthio group, an n-pentyloxymethylthio group, a 2-methoxyethylthio group, a 2-ethoxyethylthio group, a 2-n-propyloxyethylthio group, a 2-isopropyloxyethylthio group, a 2-n-butyloxyethylthio group, a 2-isobutyloxyethylthio group, a 2-tert-butyloxyethylthio group, a 2-sec-butyloxyethylthio group, a 2-n-pentyloxyethylthio group, a 2-isopentyloxyethylthio group, a 2-sec-pentyloxyethylthio group, a 2-n-hexyloxyethylthio group, a 2-(4-ethylcyclohexyloxy)ethylthio group, a 2-n-nonyloxyethylthio group, a 2-(3,5,5-trimethylhexyloxy)ethylthio group, a 2-n-decyloxyethylthio group, a 2-n-dodecyloxyethylthio group, a 3-methoxypropylthio group, a 3-ethoxypropylthio group, a 3-(n-propylthio)propylthio group, a 2-isopentyloxypropylthio group, a 2-methoxybutylthio group, a 4-ethoxybutylthio group, a 4-(n-propyloxy)butylthio group, a 5-methoxypentylthio group, a 5-ethoxypentylthio group, and a 6-n-propyloxyhexylthio group;
an alkylthio group having an alkoxyalkoxy group such as a methoxymethoxymethylthio group, an ethoxymethoxymethylthio group, a propyloxymethoxymethylthio group, a butyloxymethoxymethylthio group, an ethoxyethoxymethylthio group, a propyloxyethoxymethylthio group, a methoxymethoxyethylthio group, an ethoxymethoxyethylthio group, a propyloxymethoxyethylthio group, a butyloxymethoxyethylthio group, a propyloxyethoxyethylthio group, a butyloxyethoxyethylthio group, a propyloxybutyloxyethylthio group, a methoxymethoxypropylthio group, an ethoxymethoxypropylthio group, a butyloxymethoxypropylthio group, a butyloxymethoxybutylthio group, an ethoxyethoxybutylthio group, a cyclohexyloxyethoxyethylthio group, and a [4-(3,5,5-trimethylhexyloxy)butyloxy]ethylthio group;
an alkylthio group having an alkoxycarbonyl group such as a methoxycarbonylmethylthio group, an ethoxycarbonylmethylthio group, an n-propyloxycarbonylmethylthio group, a methoxycarbonylethylthio group, an ethoxycarbonylethylthio group, an n-propyloxycarbonylethylthio group, and an ethoxycarbonylpropylthio group;
an alkylthio group having an alkylamino group such as a methylaminomethylthio group, a 2-methylaminoethylthio group, a 2-(2-methylaminoethoxy)ethylthio group, a 4-methylaminobutylthio group, a 1-methylaminoprop-2-ylthio group, a 3-methylaminopropylthio group, a 2-ethylaminoethylthio group, a 2-(2-ethylaminoethoxy)ethylthio group, a 3-ethylaminopropylthio group, a 1-ethylaminopropylthio group, a 2-isopropylaminoethylthio group, a 2-(n-butylamino)ethylthio group, a 3-(n-hexylamino)propylthio group, and a 4-(cyclohexylamino)butylthio group;
an alkylthio group having a dialkylamino group such as a dimethylaminomethylthio group, a 2-dimethylaminoethylthio group, a 4-dimethylaminobutylthio group, a 1-dimethylaminoprop-2-ylthio group, a 3-dimethylaminopropylthio group, a 2-diethylaminoethylthio group, a 3-diethylaminopropylthio group, a 2-diisopropylaminoethylthio group, a 2-(di-n-butylamino)ethylthio group, a 2-piperidylethylthio group, and a 3-(di-n-hexylamino)propylthio group;
an alkylthio group having an alkylthio group such as a methylthiomethylthio group, a 2-methylthioethylthio group, a 2-ethylthioethylthio group, a 2-n-propylthioethylthio group, a 2-isopropylthioethylthio group, a 2-n-butylthioethylthio group, a 2-isobutylthioethylthio group, and a (3,5,5-trimethylhexylthio)hexylthio group;
an alkylthio group having a heterocyclic group such as a 2-N-morpholinylethylthio group, a 2-N-pyridylethylthio group, a 2-N-pyrrolylethylthio group, a 2-(2-furyl)ethylthio group, a 2-(1-indolyl)ethylthio group, a 2-(3-thienyl)ethylthio group, a 3-N-morpholinylpropylthio group, a 3-N-pyridylpropylthio group, a 3-N-pyrrolylpropylthio group, and a 3-(1-indolyl)propylthio group; and
an alkylthio group having an aryl group such as a phenylmethylthio group, a methylphenylmethylthio group, an ethylphenylmethylthio group, a tert-butylphenylmethylthio group, a phenylethylthio group, a methylphenylethylthio group, an ethylphenylethylthio group, and a tert-butylphenylethylthio group.

Examples of the substituted or unsubstituted arylthio group include an arylthio group having 6 to 25 carbon atoms and preferably 6 to 18 carbon atoms, and specific examples thereof include a phenylthio group, a 2-methylphenylthio group, a 4-methylphenylthio group, a 3-ethylphenylthio group, a 4-n-propylphenylthio group, a 4-isopropylphenylthio group, a 4-n-butylphenylthio group, a 4-isobutylphenylthio group, a 4-tert-butylphenylthio group, a 4-n-pentylphenylthio group, a 4-n-hexylphenylthio group, a 4-cyclohexylphenylthio group, a 4-n-octylphenylthio group, a 4-n-dodecylphenylthio group, a 4-n-octadecylphenylthio group, a 2,4-dimethylphenylthio group, a 2,5-dimethylphenylthio group, a 3,4-dimethylphenylthio group, a 2,4,6-trimethylphenylthio group, a 5-indanylthio group, a 1,2,3,4-tetrahydro-6-naphthylthio group, a 2-methoxyphenylthio group, a 3-methoxyphenylthio group, a 4-methoxyphenylthio group, a 4-ethoxyphenylthio group, a 4-n-propoxyphenylthio group, a 2,4-dimethoxyphenylthio group, a 3,5-diethoxyphenylthio group, a 2-methoxy-4-methylphenylthio group, a 2-methyl-4-methoxyphenylthio group, a 2-fluorophenylthio group, a 4-fluorophenylthio group, a 2-chlorophenylthio group, a 4-chlorophenylthio group, a 4-bromophenylthio group, a 4-trifluoromethylphenylthio group, a 3-trifluoromethylphenylthio group, a 2,4-difluorophenylthio group, a 2,4-dichlorophenylthio group, a 2,4,6-trifluorophenylthio group, a 2,4,6-trichlorophenylthio group, a 2-chloro-4-methoxyphenylthio group, a 2-naphthylthio group, a 4-methyl-1-naphthylthio group, a 4-ethoxy-1-naphthylthio group, a 2-pyridylthio group, a 4-aminophenylthio group, a 4-(N,N-dimethylamino)phenylthio group, a 4-(N,N-diethylamino)-1-naphthylthio group, a 4-[N,N-di(4'-methylphenyl)amino]phenylthio group, a 4-(N-phenoxazinyl)phenylthio group, a 4-hydroxyphenylthio group, a 2,4-dihydroxyphenylthio group, and a 4-methylthiophenylthio group.

R₃ to R₆ are optionally linked to each other to form a ring. In a case of forming a ring, usually, adjacent R₃ and R₄, R₄ and R₅, and R₅ and R₆ are linked to each other, and a ring is formed including carbon atoms to which each of R₃, R₄, R₅, and R₆ is bonded. Preferred examples of the ring formed in this way include a substituted or unsubstituted carbocyclic aliphatic ring, a substituted or unsubstituted carbocyclic aromatic ring, a substituted or unsubstituted heterocyclic aliphatic ring, and a substituted or unsubstituted heterocyclic aromatic ring, and more preferred examples thereof include a substituted or unsubstituted carbocyclic aliphatic ring having a total number of carbon atoms of 5 to 20, a substituted or unsubstituted carbocyclic aromatic ring having a total number of carbon atoms of 6 to 20, a substituted or unsubstituted heterocyclic aliphatic ring having a total number of carbon atoms of 4 to 20, and a substituted or unsubstituted heterocyclic aromatic ring having a total number of carbon atoms of 4 to 20.

Specific examples of the carbocyclic aliphatic ring include
a cyclopentane ring, a cyclohexane ring, a cyclohexene ring, a cycloheptane ring, a cyclooctane ring, and a cyclodecane ring.

Specific examples of the carbocyclic aromatic ring include
a benzene ring and a naphthalene ring.

Specific examples of the heterocyclic aliphatic ring include
a dihydrofuran ring, a tetrahydrofuran ring, a dihydrothiophene ring, a dioxane ring, and a dithiane ring.

Specific examples of the heterocyclic aromatic ring include
a furan ring, a thiophene ring, a benzofuran ring, a benzothiophene ring, and a pyrrole ring. The carbocyclic aliphatic ring, the carbocyclic aromatic ring, the heterocyclic aliphatic ring, and the heterocyclic aromatic ring to be formed may have a plurality of substituents.

Examples of the substituent include a halogen atom, a carboxyl group, a cyano group, a nitro group, a hydroxyl group, a linear, branched, or cyclic alkyl group having 1 to 20 carbon atoms, a linear, branched, or cyclic alkoxy group having 1 to 20 carbon atoms, and an aryl group which are optionally substituted with a halogen atom, a carboxyl group, a cyano group, a nitro group, a hydroxyl group, a linear, branched, or cyclic alkyl group having 1 to 20 carbon atoms, or a linear, branched, or cyclic alkoxy group having 1 to 20 carbon atoms.

From the viewpoint of the effect of the present invention, it is preferable that R₃ to R₆ shown in Formula (1) each independently represent
a hydrogen atom, a halogen atom, a substituted or unsubstituted alkyl group having 1 to 20 carbon atoms, a substituted or unsubstituted alkoxy group having 1 to 25 carbon atoms, a substituted or unsubstituted aryloxy group having 6 to 25 carbon atoms, a substituted or unsubstituted alkylthio group having 1 to 25 carbon atoms, or a substituted or unsubstituted arylthio group having 6 to 25 carbon atoms, or R₃ to R₆ are bonded to each other to form a substituted or unsubstituted carbocyclic aliphatic ring having 5 to 20 carbon atoms or a substituted or unsubstituted carbocyclic aromatic ring having 6 to 20 carbon atoms, and
it is more preferable that R₃ to R₆ each independently represent a hydrogen atom, a substituted or unsubstituted alkyl group having 1 to 20 carbon atoms, or a substituted or unsubstituted alkoxy group having 1 to 25 carbon atoms.

Examples of the unsubstituted alkyl group in R₇ shown in Formula (1) include
a methyl group, an ethyl group, an n-propyl group, an isopropyl group, an n-butyl group, an isobutyl group, a sec-butyl group, a tert-butyl group, an n-pentyl group, an isopentyl group, a neopentyl group, a sec-pentyl group, a tert-pentyl group, an n-hexyl group, a 1-methylpentyl group, a 4-methylpentyl group, a 2-ethylbutyl group, an n-heptyl group, a 1-methylhexyl group, an n-octyl group, a tert-octyl group, a 1-methylheptyl group, and a 2-ethylhexyl group.

From the viewpoint of the effect of the present invention, R₇ shown in Formula (1) each independently represent preferably a hydrogen atom or an alkyl group having 1 to 12 carbon atoms, and more preferably a hydrogen atom or an unsubstituted alkyl group having 1 to 8 carbon atoms.

Q shown in Formula (1) represents an unsubstituted benzene ring or an unsubstituted naphthalene ring.

Specifically, the unsubstituted arylene group in A shown in Formula (1) is a divalent group generated by removing one hydrogen atom from each of two different carbon atoms constituting an aryl ring, and examples thereof, depending on the position of the carbon atom to be removed, include a 1,2-phenylene group, a 1,3-phenylene group, a 1,4-phenylene group, a 1,4-naphthylene group, a 1,5-naphthylene group, a 2,7-naphthylene group, a 4,4'-biphenylene group, and a 4,4"-terphenylene group.

In a case where the arylene group is an arylene group having a substituent, the substituent is preferably a linear, branched, or cyclic alkyl group, and specific examples of the arylene group having an alkyl group include a 1,2,3,4-tetrahydro-5,8-naphthylene group, a 5-methyl-1,3-phenylene group, a 2,3-dicyclohexyl-1,4-phenylene group, and a 4-tert-butyl-1,3-phenylene group.

From the viewpoint of the effect of the present invention, in A shown in Formula (1), the substituted or unsubstituted arylene group is preferably an unsubstituted or substituted arylene group having 6 to 18 carbon atoms, and more preferably an unsubstituted or substituted arylene group having 6 to 14 carbon atoms.

In the group represented by General Formula (2), Z represents an oxygen atom, a sulfur atom, a phenylene group, a ketone group, or a substituted or unsubstituted alkylene group.

As described above, the phenylene group is a divalent group generated by removing one hydrogen atom from each of two different carbon atoms constituting a benzene ring, and preferred examples thereof include a 1,4-phenylene group.

The ketone group means a group represented by [-(C=O)-] (neither of both ends is bonded to a hydrogen atom) in the present invention.

The alkylene group is a divalent group generated by removing one hydrogen atom from each of two different carbon atoms constituting a substituted or unsubstituted alkyl group, and preferably has 1 to 20 carbon atoms, more preferably 1 to 15 carbon atoms.

Examples of the substituted or unsubstituted alkyl group include the specific examples described above for R₁ and R₂.

Examples of the unsubstituted alkylene group consisting of an unsubstituted alkyl group include linear alkylene groups consisting of only carbon atoms and hydrogen atoms, such as a methylene group [-CH₂-], an ethylene group [-CH₂-CH₂-], an n-propylene group [-CH₂-CH₂-CH₂-], and an n-butylene group [-CH₂-CH₂-CH₂-CH₂-]; and
branched alkylene groups consisting of only carbon atoms and hydrogen atoms, such as a 1,2-dimethylethylene group [-CH(CH₃)-CH(CH₃)-], an ethylidene group [-CH(CH₃)-], and an isopropylidene group [-C(CH₃)₂-].

Examples of the substituted alkylene group consisting of a substituted alkyl group include branched substituted alkylene groups having a halogen atom, such as a ditrifluoromethylmethylene group [-C(CF₃)₂-]; and
monocyclic or bridged alicyclic alkylene groups such as a 1,3-cyclopentylene group, a 1,4-cyclohexylene group, a 1,2-cyclohexylene group, a 2,3-bicyclo[2.2.1]heptylene group, a 2,5-bicyclo[2.2.1]heptylene group, a 2,6-bicyclo[2.2.1]heptylene group, a 2,6-bicyclo[2.2.2]octylene group, and a 1,3-adamantylene group.

In R₈, examples of the unsubstituted alkyl group include the specific examples of the linear unsubstituted alkyl group described above for R₁ and R₂, and an alkyl group having 1 to 4 carbon atoms is preferable.

l and m each independently represent 0 or 1. However, in a case where l is 0, m represents 1, and in a case where m is 0, l represents 1.

From the viewpoint of the effects of the present invention, A shown in the Formula (1) is preferably a substituted or unsubstituted arylene group having 6 to 18 carbon atoms or a group represented by General Formula (2) (in the formula, Z represents an oxygen atom, a sulfur atom, a ketone group, or a substituted or unsubstituted alkylene group having 1 to 20 carbon atoms, and R₈ represents a hydrogen atom or an alkyl group having 1 to 4 carbon atoms), and
more preferably a substituted or unsubstituted arylene group having 6 to 14 carbon atoms.

n shown in the Formula (1) represents 0 or 1. However, in a case where n is 0, it represents that they are directly bonded without interposing A to form a fluoran dimer compound. In the present invention, it is particularly preferable that n is 0.

Preferred aspects of the fluoran dimer compound according to the present invention are represented by General Formulae (1-1) to (1-10), but the present invention is not limited thereto.

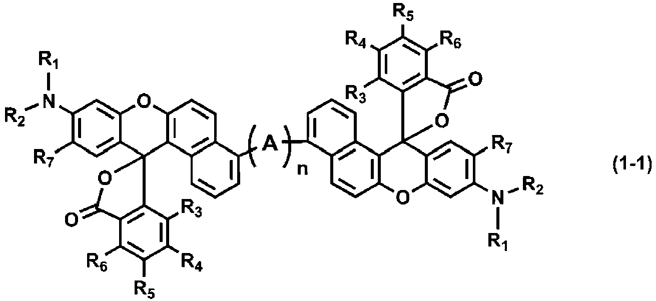

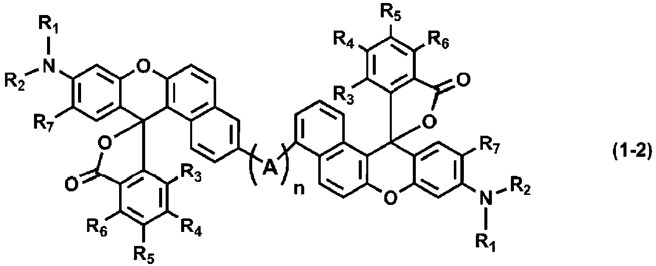

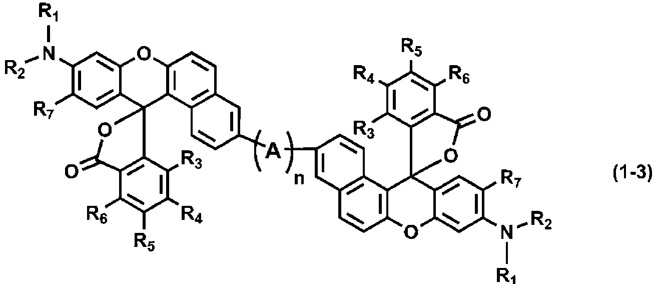

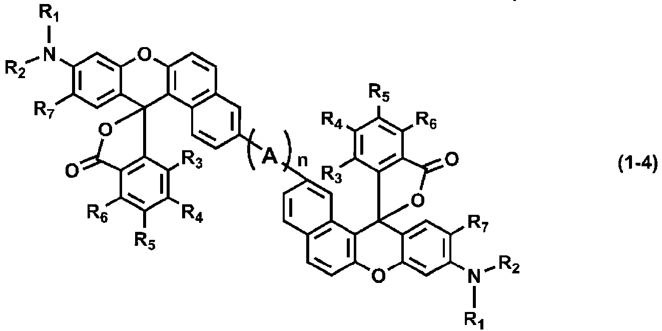

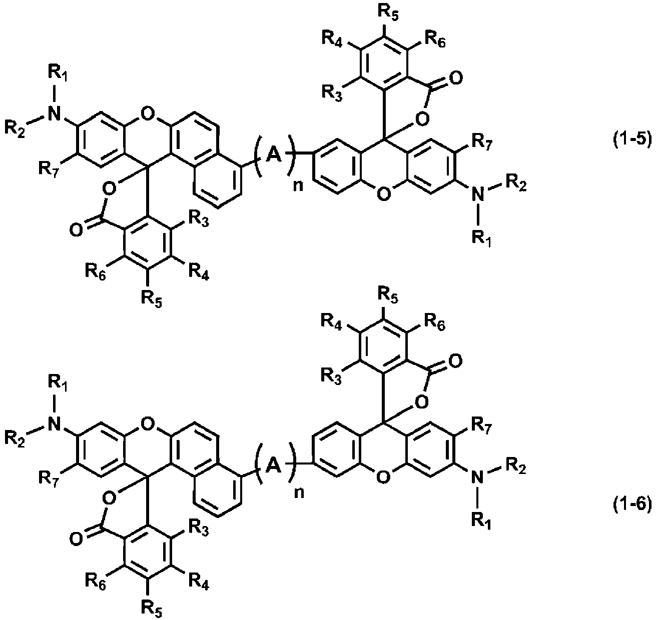

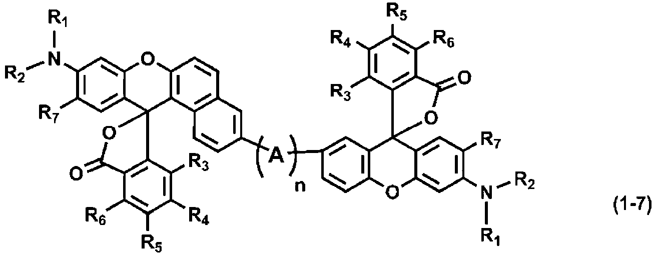

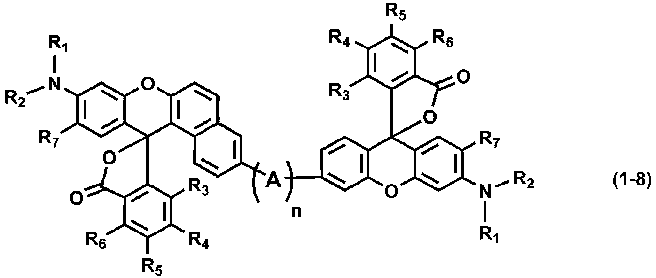

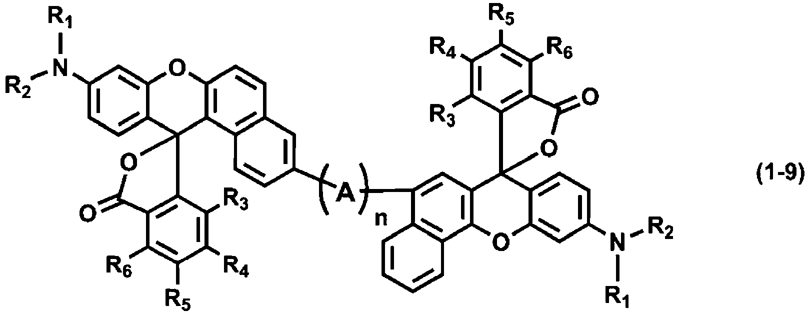

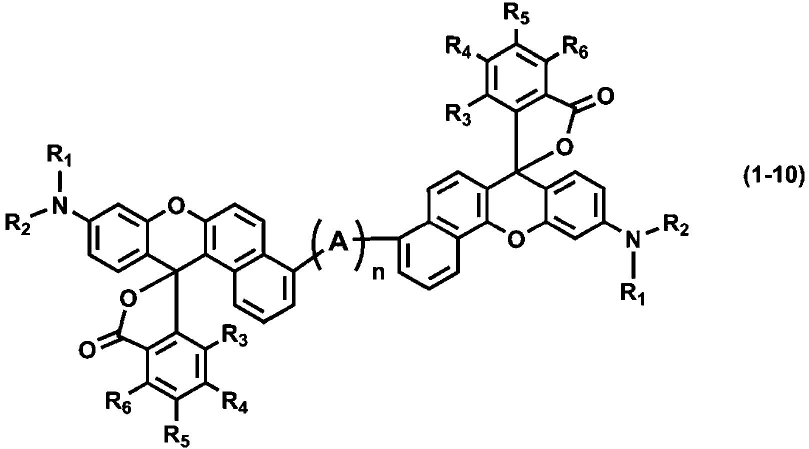

R₁ to R₇, A, and n in General Formulae (1-1) to (1-10) have the same meanings as the examples of R₁ to R₇, A, and n in General Formula (1), and the same applies to the preferred aspects thereof.

Hereinafter, specific examples of the fluoran dimer compound represented by General Formula (1) include the following compounds, but the present invention is not limited thereto.

In the table, "-" represents a case where n in General Formulae (1-1) to (1-10) is 0, and represents that the fluoran dimer compound is formed by direct bonding without interposing A.

| Compound Number | General Formula | R1 | R2 | A | R3 | R4 | R5 | R6 | R7 |
|---|---|---|---|---|---|---|---|---|---|
| 1 | 1-3 | n-butyl group | n-butyl group | - | H | H | H | H | H |
| 2 | 1-3 | n-octyl group | n-octyl group | - | H | H | H | H | H |
| 3 | 1-3 | n-propyl group | Cyclohexyl group | - | H | H | H | H | H |
| 4 | 1-3 | n-butyl group | 3-methoxypropyl group | - | H | H | H | H | H |
| 5 | 1-3 | Ethyl group | 4-methylphenyl group | - | H | H | H | H | H |
| 6 | 1-3 | n-propyl group | Ethoxyethyl group | - | H | H | H | H | H |
| 7 | 1-3 | Ethyl group | Tetrahydrofuranyl methyl group | - | H | H | H | H | H |
| 8 | 1-3 | Piperidine ring | | - | H | H | H | H | H |
| 9 | 1-3 | Ethoxyethyl group | Ethoxyethyl group | - | H | H | H | H | H |
| 10 | 1-3 | Morpholine ring | | - | H | H | H | H | H |
| 11 | 1-3 | n-butyl group | n-butyl group | 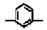 | H | H | H | H | H |
| 12 | 1-3 | Ethyl group | Ethyl group | 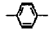 | H | Methyl group | H | H | H |
| 13 | 1-3 | Ethoxyethyl group | Ethoxyethyl group | 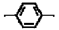 | H | H | H | H | H |
| 14 | 1-3 | Ethyl group | Isopentyl group | 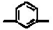 | H | H | H | H | H |
| 15 | 1-3 | Methyl group | Cyclohexyl group | 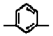 | H | H | H | H | H |

| Compound Number | General Formula | R1 | R2 | A | R3 | R4 | R5 | R6 | R7 |
|---|---|---|---|---|---|---|---|---|---|
| 16 | 1-3 | n-butyl group | 3- methoxypropyl group | 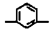 | H | H | H | H | H |
| 17 | 1-3 | n-butyl group | n-butyl group | 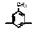 | H | H | H | H | H |
| 18 | 1-3 | n-butyl group | Benzyl group | 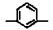 | H | H | H | H | H |
| 19 | 1-3 | Methyl group | sec-pentyl group | 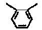 | H | H | H | H | H |
| 20 | 1-3 | n-butyl group | n-butyl group | 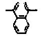 | H | H | H | H | H |
| 21 | 1-3 | n-propyl group | (2-methoxyethoxy)ethyl group | 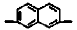 | H | H | H | H | H |
| 22 | 1-1 | n-butyl group | n-butyl group | 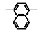 | H | H | H | H | H |
| 23 | 1-3 | Tetrahydrofuranyl methyl group | Tetrahydrofuranyl methyl group | - | H | H | H | H | H |
| 24 | 1-1 | n-butyl group | n-butyl group | 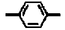 | H | H | H | H | H |
| 25 | 1-1 | n-butyl group | Benzyl group | 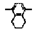 | H | H | H | H | H |
| 26 | 1-1 | Methyl group | sec-pentyl group | 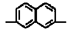 | H | H | H | H | H |
| 27 | 1-3 | n-butyl group | n-butyl group | 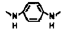 | H | H | H | H | H |

| Compound Number | General Formula | R1 | R2 | A | R3 | R4 | R5 | R6 | R7 |
|---|---|---|---|---|---|---|---|---|---|
| 28 | 1-3 | n-propyl group | (2-methoxyethoxy)ethyl group | 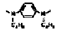 | H | H | H | H | H |
| 29 | 1-3 | Ethyl group | Ethyl group | 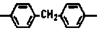 | H | H | H | H | H |
| 30 | 1-3 | n-butyl group | n-butyl group | 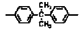 | H | H | H | H | H |
| 31 | 1-3 | Tetrahydrofuranyl methyl group | Tetrahydrofuranyl methyl group | 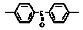 | H | H | H | H | H |
| 32 | 1-3 | Methyl group | sec-pentyl group | 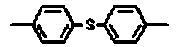 | H | H | H | H | H |
| 33 | 1-3 | n-propyl group | n-propyl group | 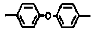 | H | H | H | H | H |
| 34 | 1-3 | n-propyl group | (2-methoxyethoxy)ethyl group | 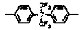 | H | H | H | H | H |
| 35 | 1-3 | Ethyl group | Ethyl group | - | H | tert-butyl group | H | H | H |
| 36 | 1-3 | Ethyl group | Ethyl group | - | H | n-Pentyloxy group | H | H | H |
| 37 | 1-3 | Ethyl group | Ethyl group | - | Ethoxy group | H | H | Ethoxy group | H |
| 38 | 1-3 | Ethyl group | Ethyl group | - | Ethoxy group | -Cl | -Cl | Ethoxy group | H |
| 39 | 1-3 | Ethoxyethyl group | Ethoxyethyl group | - | H | H | tert-butyl group | H | H |
| 40 | 1-3 | Ethyl group | Ethoxyethyl group | - | H | H | Isopropyl group | H | H |

| Compound Number | General Formula | R1 | R2 | A | R3 | R4 | R5 | R6 | R7 |
|---|---|---|---|---|---|---|---|---|---|
| 41 | 1-3 | Ethyl group | Ethyl group | 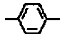 | Ethoxy group | H | H | Ethoxy group | H |
| 42 | 1-3 | Ethyl group | Ethyl group | 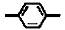 | H | tert-butyl group | H | H | H |
| 43 | 1-3 | Ethyl group | Ethyl group | 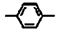 | H | n-pentyloxy group | H | H | H |
| 44 | 1-3 | Ethyl group | Isopentyl group | 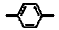 | H | Methyl group | Methyl group | H | H |
| 45 | 1-3 | Ethyl group | Ethyl group | 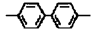 | H | H | H | H | H |
| 46 | 1-3 | Ethyl group | Ethyl group | 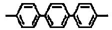 | H | n-pentyloxy group | H | H | H |
| 47 | 1-7 | Ethyl group | Ethyl group | - | H | H | H | H | H |
| 48 | 1-7 | Ethoxyethyl group | Ethoxyethyl group | - | H | H | H | H | H |
| 49 | 1-7 | Methyl group | 4-methylpentyl group | - | H | H | H | H | H |
| 50 | 1-7 | Ethyl group | Ethyl group | - | H | n-pentyloxy group | H | H | H |
| 51 | 1-5 | n-butyl group | n-butyl group | - | H | H | H | H | H |
| 52 | 1-5 | Ethoxyethyl group | Ethoxyethyl group | - | H | H | H | H | H |
| 53 | 1-5 | n-butyl group | n-butyl group | - | H | H | tert-pentyl group | H | H |
| 54 | 1-5 | n-butyl group | n-butyl group | - | H | Phenyloxy group | H | H | H |
| 55 | 1-7 | n-propyl group | n-propyl group | 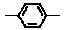 | H | H | H | H | H |

| Compound Number | General Formula | R1 | R2 | A | R3 | R4 | R5 | R6 | R7 |
|---|---|---|---|---|---|---|---|---|---|
| 56 | 1-7 | Methyl group | Cyclohexyl group | 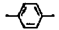 | H | H | H | H | H |
| 57 | 1-7 | Ethyl group | Tetrahydrofuranyl methyl group | 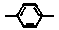 | H | Isopropyl group | H | H | H |
| 58 | 1-5 | Piperidine ring | | 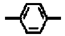 | H | H | Isopentyloxy group | H | H |
| 59 | 1-5 | Ethyl group | 4-methylphenyl group | 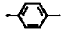 | H | n-butyloxy group | H | H | H |
| 60 | 1-5 | Methyl group | sec-pentyl group | 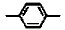 | H | Methyl group | Methyl group | H | H |
| 61 | 1-3 | Ethyl group | Ethyl group | - | n-pentyloxy group | -Cl | -Cl | n-pentyloxy group | H |
| 62 | 1-3 | Ethoxyethyl group | Ethoxyethyl group | - | n-propyloxy group | Methylthio group | Methylthio group | n-propyloxy group | H |
| 63 | 1-3 | n-butyl group | n-butyl group | - | H | Cyclohexane ring | | H | H |
| 64 | 1-3 | Ethoxyethyl group | Ethoxyethyl group | - | H | Benzene ring | | H | H |
| 65 | 1-3 | Ethyl group | Ethyl group | 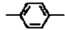 | H | Cyclohexane ring | | H | H |
| 66 | 1-3 | Ethyl group | Ethyl group | 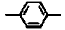 | H | Benzene ring | | H | H |
| 67 | 1-7 | n-propyl group | 4-methylpentyl group | 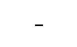 | H | Benzene ring | | H | H |
| 68 | 1-7 | n-propyl group | n-propyl group | 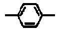 | H | Isopentylthio group | H | H | H |
| 69 | 1-5 | Ethyl group | 4-methylphenyl group | 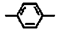 | H | 4-tert-butylphenylthio group | H | H | H |
| 70 | 1-5 | Methyl group | sec-pentyl group | 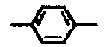 | H | Ethylthio group | Ethylthio group | H | H |

| Compound Number | General Formula | R1 | R2 | A | R3 | R4 | R5 | R6 | R7 |
|---|---|---|---|---|---|---|---|---|---|
| 71 | 1-3 | Ethyl group | Ethyl group | 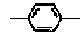 | H | H | H | H | H |
| 72 | 1-3 | (2-methoxyethoxy)ethyl group | (2-methoxyethoxy)ethyl group | - | H | H | H | H | H |
| 73 | 1-3 | Ethyl group | Ethyl group | - | H | H | H | H | H |
| 74 | 1-3 | (2-methoxyethoxy)ethyl group | (2-methoxyethoxy)ethyl group | - | H | tert-butyl group | H | H | H |
| 75 | 1-3 | n-butyl group | n-butyl group | - | H | tert-butyl group | H | H | H |
| 76 | 1-3 | Ethyl group | 4-methylphenyl group | - | H | tert-butyl group | H | H | H |
| 77 | 1-3 | Ethyl group | Ethyl group | 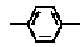 | H | tert-octyl group | H | H | H |
| 78 | 1-7 | n-propyl group | 4-methylphenyl group | - | H | tert-butyl group | H | H | H |
| 79 | 1-3 | Ethyl group | Ethyl group | - | H | H | Methyl group | H | H |
| 80 | 1-3 | n-butyl group | n-butyl group | - | H | H | Methyl group | H | H |
| 81 | 1-3 | Ethyl group | Ethyl group | - | H | -F | H | H | H |
| 82 | 1-3 | n-butyl group | n-butyl group | - | H | -F | H | H | H |
| 83 | 1-3 | Ethyl group | Ethyl group | - | H | -F | -F | H | H |
| 84 | 1-3 | n-butyl group | n-butyl group | - | H | -F | -F | H | H |
| 85 | 1-3 | Ethyl group | Ethyl group | - | H | Trifluoromethyl group | H | H | H |

| Compound Number | General Formula | R1 | R2 | A | R3 | R4 | R5 | R6 | R7 |
|---|---|---|---|---|---|---|---|---|---|
| 86 | 1-3 | n-butyl group | n-butyl group | - | H | Trifluoromethyl group | H | H | H |
| 87 | 1-3 | n-hexyl group | -CH₂CF₂CH₂CF₃ | - | H | tert-butyl group | H | H | H |
| 88 | 1-3 | n-hexyl group | -CH₂CF₂CH₂CF₃ | - | H | Methyl group | H | H | H |
| 89 | 1-3 | Ethyl group | 2-trifluoromethylphenyl group | - | H | tert-butyl group | H | H | H |
| 90 | 1-3 | Ethyl group | 2-trifluoromethylphenyl group | - | H | Methyl group | H | H | H |
| 91 | 1-7 | Ethyl group | Ethyl group | - | H | tert-butyl group | H | H | H |
| 92 | 1-7 | Ethyl group | Ethyl group | - | H | H | Methyl group | H | H |
| 93 | 1-7 | n-butyl group | n-butyl group | - | H | tert-butyl group | H | H | H |
| 94 | 1-7 | n-butyl group | n-butyl group | - | H | H | Methyl group | H | H |
| 95 | 1-7 | n-hexyl group | -CH₂CF₂CH₂CF₃ | - | H | tert-butyl group | H | H | H |
| 96 | 1-7 | n-hexyl group | -CH₂CF₂CH₂CF₃ | - | H | Methyl group | Methyl group | H | H |
| 97 | 1-7 | Ethyl group | 2-trifluoromethylphenyl group | - | H | tert-butyl group | H | H | H |
| 98 | 1-7 | Ethyl group | 2-trifluoromethylphenyl group | - | H | Methyl group | H | H | H |
| 99 | 1-7 | Ethyl group | 2-fluorophenyl group | - | H | tert-butyl group | H | H | H |
| 100 | 1-7 | Ethyl group | 2-fluorophenyl group | - | H | Methyl group | H | H | H |

| Compound Number | General Formula | R1 | R2 | A | R3 | R4 | R5 | R6 | R7 |
|---|---|---|---|---|---|---|---|---|---|
| 101 | 1-4 | Ethyl group | Ethyl group | - | H | H | H | H | H |
| 102 | 1-4 | n-butyl group | n-butyl group | - | H | H | H | H | H |
| 103 | 1-8 | Ethyl group | Ethyl group | - | H | H | H | H | H |
| 104 | 1-8 | n-butyl group | n-butyl group | - | H | H | H | H | H |
| 105 | 1-4 | Ethyl group | Ethyl group | - | H | tert-butyl group | H | H | H |
| 106 | 1-4 | Ethyl group | Ethyl group | - | H | Methyl group | H | H | H |
| 107 | 1-8 | n-butyl group | n-butyl group | - | H | tert-butyl group | H | H | H |
| 108 | 1-8 | n-butyl group | n-butyl group | - | H | Methyl group | H | H | H |
| 109 | 1-4 | Ethyl group | Isopentyl group | - | H | tert-butyl group | H | H | H |
| 110 | 1-8 | Ethyl group | sec-butyl group | - | H | H | Methyl group | H | H |
| 111 | 1-2 | Ethyl group | Ethyl group | - | H | H | H | H | H |
| 112 | 1-1 | Ethyl group | Ethyl group | - | H | tert-butyl group | H | H | H |
| 113 | 1-2 | n-butyl group | n-butyl group | - | H | H | H | H | H |
| 114 | 1-1 | n-butyl group | n-butyl group | - | H | H | Methyl group | H | H |
| 115 | 1-5 | Ethyl group | Ethyl group | - | H | tert-butyl group | H | H | H |

| Compound Number | General Formula | R1 | R2 | A | R3 | R4 | R5 | R6 | R7 |
|---|---|---|---|---|---|---|---|---|---|
| 116 | 1-6 | Ethyl group | Ethyl group | - | H | Methyl group | H | H | H |
| 117 | 1-5 | n-butyl group | n-butyl group | - | H | tert-butyl group | H | H | H |
| 118 | 1-6 | n-butyl group | n-butyl group | - | H | H | H | H | H |
| 119 | 1-5 | Ethyl group | 4-methylphenyl group | - | H | H | H | H | H |
| 120 | 1-1 | CH₂CF₂CF₃ | -CH₂CF₂CF₃ | - | H | tert-butyl group | H | H | H |
| 121 | 1-3 | Ethyl group | H | - | H | tert-butyl group | H | H | Methyl group |
| 122 | 1-3 | n-hexyl group | H | - | H | tert-butyl group | H | H | H |
| 123 | 1-9 | Ethyl group | Ethyl group | - | H | tert-butyl group | H | H | H |
| 124 | 1-9 | n-butyl group | n-butyl group | - | H | tert-butyl group | H | H | H |
| 125 | 1-9 | Ethyl group | Ethyl group | - | H | H | Methyl group | H | H |
| 126 | 1-9 | n-butyl group | n-butyl group | - | H | H | Methyl group | H | H |
| 127 | 1-10 | Ethyl group | Ethyl group | - | H | tert-butyl group | H | H | H |
| 128 | 1-10 | n-butyl group | n-butyl group | - | H | tert-butyl group | H | H | H |
| 129 | 1-9 | Ethyl group | Ethyl group | 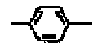 | H | tert-butyl group | H | H | H |
| 130 | 1-9 | n-butyl group | n-butyl group | 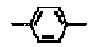 | H | tert-butyl group | H | H | H |

### [Manufacturing method of fluoran dimer compound]

The fluoran dimer compound represented by General Formula (1) can be manufactured by a known method. For example, the fluoran dimer compound can be manufactured by the method described in Japanese Unexamined Patent Publication No. H5-70701.

In the present embodiment, a method for manufacturing the fluoran dimer compound of General Formula (1-3) will be described with the following reaction formula as an example.

In this method, the compound can be manufactured by reacting a keto acid derivative represented by Formula (3) which can be manufactured by, for example, the method described in Japanese Unexamined Patent Publication No. H9-20734, and a dimethoxybisnaphthalene derivative represented by Formula (4) which can be manufactured by a reaction of cross-coupling an organic boron compound and an organic halogen compound in the presence of a palladium catalyst described in, for example, Japanese Unexamined Patent Publication No. 2023-182866,
at a temperature of 0°C to 100°C for several tens of hours in the presence of a dehydrating condensing agent, and treating the product with an alkali.

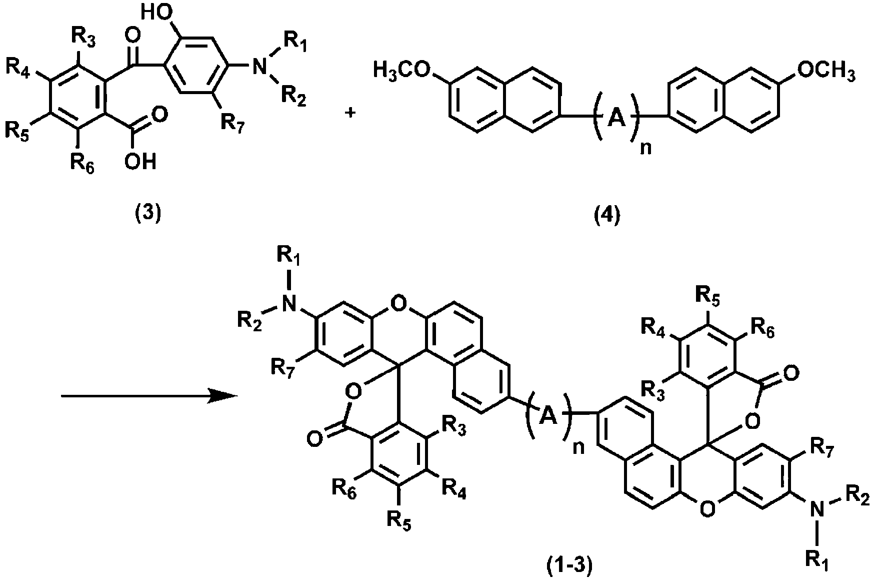

In Formula (3) and Formula (1-3), R₁ to R₇ have the same meanings as R₁ to R₇ in General Formula (1), and in Formula (4) and Formula (1-3), A and n have the same meanings as A and n in General Formula (1).

The reaction between the keto acid derivative represented by Formula (3) and the bisnaphthalene derivative having a naphthalene ring of Formula (4) can be performed in the air, and as the dehydrating condensing agent, concentrated sulfuric acid, fuming sulfuric acid, polyphosphoric acid, or phosphorus pentoxide can be used, but concentrated sulfuric acid is particularly preferable. In a case where concentrated sulfuric acid is used as the dehydrating condensing agent, the reaction temperature is preferably in a range of 0°C to 180°C, and is particularly preferably 0°C to 120°C.

The treatment with an alkali is to react the above-described keto acid derivative represented by Formula (3) and the bisnaphthalene derivative of Formula (4) in the presence of a dehydrating condensing agent, and then to place the product under an alkali condition at a certain temperature for a certain period of time. As the alkali, sodium hydroxide, potassium hydroxide, sodium carbonate, or the like can be used, but sodium hydroxide is particularly preferable, and it is preferable to use it as an aqueous solution.

The temperature of the treatment with an alkali is 0°C to 100°C, preferably 50°C to 100°C, but in general, the higher the temperature, the more efficiently the treatment proceeds. The alkali is preferably used in such an amount that the treatment liquid has a pH of 9 or more. The reactant treated with an alkali is extracted with an organic solvent and purified.

In addition, an organic solvent may be allowed to coexist during the alkali treatment. As the organic solvent, benzene, toluene, xylene, chlorobenzene, or the like can be used, but toluene is usually preferably used. In a case where a target substance is precipitated from an organic solvent, methanol, ethanol, n-propanol, and isopropanol may be used in combination.

The fluoran dimer compound represented by General Formula (1) produced in this way may often form a solvate with a solvent (for example, an alcohol-based solvent such as methanol or isopropanol, a ketone-based solvent such as acetone or methyl ethyl ketone, or an aromatic hydrocarbon-based solvent such as benzene, toluene, or xylene), but the fluoran dimer compound represented by General Formula (1) of the present invention includes such a solvate.

In addition, the fluoran dimer compound represented by General Formula (1) may often have a plurality of crystal types (polymorphism) in a crystalline state, but the fluoran dimer compound represented by General Formula (1) of the present invention includes such polymorphism.

### [Recording Material]

The present invention is a recording material which develops a magenta color (crimson) by using a color-forming reaction between an electron-donating color forming agent and an electron-accepting color developer, and which contains, as the electron-donating color forming agent, at least one selected from fluoran dimer compounds represented by General Formula (1). The recording material is preferably a pressure-sensitive recording material or a thermosensitive recording material, and more preferably a thermosensitive recording material.

Furthermore, the present invention is a reversible thermosensitive recording material which is a reversible recording material using a color forming and decoloring reaction between an electron-donating color forming agent and a color developing/decoloring agent, and which contains, as the electron-donating color forming agent, at least one selected from fluoran dimer compounds represented by General Formula (1).

In the recording material of the present invention, at least one selected from fluoran dimer compounds represented by General Formula (1) is used as a color forming agent, but may be used in combination with other color forming agents as desired to the extent that the magenta color (crimson) is not impaired. As such other color forming agents, various known color forming agents used in recording materials can be appropriately used.

As a recording method, a developed color image can be recorded on the recording material of the present invention using any of a thermal head or a laser light as an image recording means.

The thermal head can be appropriately selected depending on the application without any limitation on the shape, structure, and size thereof. As the laser light, a laser light having a wavelength in a near-infrared region, such as a semiconductor laser, a YAG laser, or a fiber laser light, can be selected without any limitation depending on the purpose.

The reason why the developed color image of the recording material having the fluoran dimer compound of the present invention has excellent light fastness is that, in general, a coloring agent is usually excited by light to enter an activated state in which a photofading reaction occurs, but it is considered that the durability is improved by quickly deactivating the excited state to a ground state.

It is considered that, since the fluoran dimer compound of the present invention has a structure in which two fluoran derivatives are linked, the coloring agent molecules are in the form of coloring agent particles, that is, in an associated state, and the relaxation to the ground state is promoted.

Furthermore, by linking two fluoran derivatives, the solubility in oil and plasticizer is lower than that of a general fluoran compound, and the developed color image has excellent oil resistance and plasticizer resistance.

### [Thermosensitive Recording Material]

The thermosensitive recording material of the present invention can be produced by a known method without using a special method. For example, the thermosensitive recording material can be produced by various known methods disclosed in Japanese Examined Patent Publication No. S45-14039 and the like.

In general, in the presence of water, the fluoran dimer compound of the present invention, the color developer, and optionally a thermofusible compound (sensitizer) or the like are mixed together or separately, and usually, the mixture is pulverized and dispersed to a particle diameter of 3 µm or less, preferably 2 µm or less, by a mixing and pulverizing machine such as a ball mill, a sand mill (vertical or horizontal), an attritor, a colloidal mill, or the like, and the coating liquid for a thermosensitive recording layer can be prepared. In addition, in some cases, a coating liquid for a thermosensitive recording layer may be prepared by preparing a co-melting product with the fluoran dimer compound or the color developer of the present invention in advance, and dispersing the co-melting product.

In general, the coating liquid is prepared in the presence of a binder. The binder is prepared so as to be about 5% to 50% by weight of the total solid content.

As the binder, a water-soluble polymer and a water-insoluble polymer can be used. Preferred specific examples thereof include, as the water-soluble polymer, methyl cellulose, carboxymethyl cellulose, hydroxyethyl cellulose, starches, a styrene maleic anhydride copolymer hydrolysate, an isobutylene maleic anhydride copolymer hydrolysate, polyvinyl alcohol, a carboxy-modified polyvinyl alcohol, polyacrylamide, and the like, and as the water-insoluble polymer, a styrene-butadiene rubber latex, an acrylonitrile-butadiene rubber latex, a vinyl acetate emulsion, and the like, but the present invention is not limited thereto.

Further, as necessary, a pigment, a metal soap, a wax, a surfactant, an antioxidant, an ultraviolet absorber, an ultraviolet stabilizer, an antifoaming agent, and the like are added to the coating liquid.

As the pigment, an organic pigment and an inorganic pigment can be used. Preferred examples thereof include an inorganic pigment such as calcium carbonate, barium carbonate, magnesium carbonate, zinc carbonate, zinc oxide, aluminum oxide, or titanium oxide, and an organic pigment such as a styrene microball, nylon particles, a urea-formalin filler, polyethylene particles, a cellulose filler, or starch particles.

As the metal soap, a metal salt of a higher fatty acid is used. Preferred specific examples thereof include zinc stearate, calcium stearate, aluminum stearate, and the like.

Examples of the wax include paraffin wax, carboxy-modified paraffin wax, polyethylene wax, and the like.

Examples of the surfactant (dispersant) include a sulfosuccinic acid-based alkali metal salt [for example, a sodium salt of di(n-hexyl) sulfosuccinic acid or di(2-ethylhexyl) sulfosuccinic acid], a sodium salt of dodecylbenzenesulfonic acid, a sodium salt of lauric alcohol sulfuric acid ester, a fatty acid metal salt, a fluorine-containing surfactant, and the like.

As the antioxidant, hindered phenols are used.

Examples of the ultraviolet absorber and the ultraviolet stabilizer include a cinnamic acid derivative, a benzophenone derivative, a triazole derivative, a salicylic acid derivative, a hindered amine derivative, and the like.

The method for forming the thermosensitive recording layer in the thermosensitive recording material according to the present invention is not particularly limited, and the thermosensitive recording layer can be formed according to a known technique. For example, a coating liquid for the thermosensitive recording layer can be applied onto a support with an appropriate coating device such as an air knife coater, a blade coater, a bar coater, a short dwell coater, a gravure coater, a curtain coater, or a Meyer bar, and dried to form a recording layer.

The coating amount of the coating liquid is not particularly limited, but is generally adjusted to about 1.5 to 12 g/m² and preferably about 2.5 to 10 g/m² in terms of dry weight.

The support is not particularly limited, and for example, paper, a plastic sheet, synthetic paper, a composite sheet obtained by combining these, a nonwoven sheet, a molded article, or a metal vapor deposited product is used.

A protective layer (overcoat layer) can be provided on the front surface and/or the back surface of the thermosensitive recording layer as necessary, and an undercoat layer consisting of a single layer or a plurality of layers containing a pigment (for example, kaolin or calcium carbonate) or a synthetic resin (for example, plastic spherical particles or plastic spherical hollow particles) and the like can be provided between the support and the thermosensitive recording layer.

In addition, it is also possible to provide an intermediate layer consisting of a pigment, a binder, and the like between the thermosensitive recording layer and the undercoat layer or between the thermosensitive recording layer and the protective layer, and various known techniques in the manufacturing method of the thermosensitive recording material, such as performing pressure-sensitive adhesive processing on the back surface of the support to process the thermosensitive recording material into a pressure-sensitive adhesive label, can be adopted.

In a case where an image recording means using laser light is used as the recording method, it is preferable to provide a photothermal conversion layer.

The photothermal conversion layer contains at least a coloring agent or a pigment (hereinafter, referred to as a photothermal conversion material) having absorption in a near-infrared region, which has a role of absorbing laser light having a wavelength in the near-infrared region with high efficiency and generating heat, and the formation method thereof is not particularly limited. The photothermal conversion layer can be formed according to a known technique, but it is used in combination with a binder resin.

The photothermal conversion material may be contained in at least one of the front surface or the back surface of the thermosensitive recording layer, and in a case where the photothermal conversion material is contained in the thermosensitive recording layer, the thermosensitive recording layer also serves as the photothermal conversion layer.

In addition, a barrier layer may be formed between the thermosensitive recording layer and the photothermal conversion layer for the purpose of suppressing the interaction between the constituent materials of the two layers, and it is preferable to contain, as a material, a resin that can be cured by heat, ultraviolet rays, electron beams, or the like. The layer sandwiched between the thermosensitive recording layer and the photothermal conversion layer is not particularly limited and can be appropriately selected according to the purpose.

Examples of the photothermal conversion material include a cyanine-based coloring agent, a croconium-based coloring agent, a polymethine-based coloring agent, an azulenium-based coloring agent, a squarylium-based coloring agent, a thiopyrylium-based coloring agent, a naphthoquinone-based coloring agent, an anthraquinone-based coloring agent, a phthalocyanine-based coloring agent, a naphthalocyanine-based coloring agent, an azo-based coloring agent, a thioamide-based coloring agent, a dithiol-based coloring agent, and an indoaniline-based coloring agent.

As the color developer used in the thermosensitive recording material, various phenolic compounds can be used. Specific examples thereof include bisphenol A, 2,2-bis(p-hydroxyphenyl)-4-methylpentane, 1,1-bis(p-hydroxyphenyl)cyclohexane, bisphenol S, 4-hydroxy-4'-isopropoxydiphenylsulfone, 3,3-diallyl-4,4'-dihydroxydiphenylsulfone, 1,5-bis(p-hydroxyphenylmercapto)-3-oxapentane, p-hydroxybenzoic acid benzyl, tetrabromobisphenol A, and tetrabromobisphenol S, and bisphenol A is particularly preferably used.

Examples of the sensitizer used in the thermosensitive recording material include p-benzylbiphenyl, meta-terphenyl, 2-benzyloxy naphthalene, 1,4-dibenzyl oxy naphthalene, benzyl oxalate, di-p-methylbenzyl oxalate, di-p-chlorobenzyl oxalate, 1,2-diphenoxy ethane, 1,2-m-toluoxy ethane, 1,2-di-p-toluoxy ethane, 1,4-diphenoxy ethane, p-benzyloxy benzoic acid benzyl, 1-hydroxy-2-naphthoic acid phenyl, and benzyl terephthalate, and p-benzylbiphenyl, metatolyl, 2-benzyloxy naphthalene, di-p-methylbenzyl oxalate, and 1,2-m-toluoxy ethane are particularly preferably used.

### [Reversible Thermosensitive Recording Material]

The reversible thermosensitive recording material according to the present invention can be produced by a known method without depending on a special method.

In general, a dispersion liquid is produced by dispersing the fluoran dimer compound of the present invention as a color forming agent, and a color-developing/decoloring agent or a long-chain color developer, respectively, together with a binder using a sand mill or the like.

These dispersion liquids are mixed, and as necessary, a basic compound for causing decolorization at a lower temperature, a thickener as a liquid property improver, a white pigment, or the like is added, and the obtained coating liquid for recording is applied to a support such as paper, a plastic film, or a sheet and then dried to obtain the reversible thermosensitive recording material.

Further, as necessary, a protective layer, a UV protective layer, or a protective layer for abrasion resistance may be provided on the recording layer for the purpose of improving thermal head matching properties or imparting durability to the recording layer.

In a case where the color-developing/decoloring agent is used, the principle is used that the lactone ring of the fluoran dimer compound of the present invention is cleaved to develop a color when in contact with a color-developing group, but is closed to be decolorized when in contact with a decolorizing group by controlling the thermal energy.

In a case where the long-chain color developer is used, in a case of being heated to a temperature equal to or higher than the melting point of the color developer, the long-chain color developer is melted and comes into contact with the fluoran dimer compound according to the present invention to be colored, and in a case of being slowly cooled from this state, the fluoran dimer compound according to the present invention and the color developer are separated and crystallized, and thus the color is decolorized.

On the other hand, in a case of being quenched, the color developer is aggregated with regularity while maintaining the bond with the fluoran dimer compound according to the present invention, and thus the color development state is maintained.

In addition, in a case of being further heated from the color development state, the aggregation structure of the color development state is broken, and when the temperature becomes lower than the color developing temperature, the color developer is in a stable crystalline state alone, and is separated from the fluoran dimer compound according to the present invention to be decolorized.

Examples of the color-developing/decoloring agent include an amphoteric compound having at least one phenolic hydroxyl group and at least one carboxyl group and having an amino group as a functional group or as a part of a salt compound, and a salt or complex salt of a compound having at least one phenolic hydroxyl group and/or carboxyl group and an aliphatic amine. Examples thereof include aliphatic amines, aminobenzoic acids, hydroxyaminobenzoic acids, or ester compounds thereof.

Examples of the long-chain color developer include a compound having a group having a color-developing property with respect to the fluoran dimer compound according to the present invention, for example, a phenolic hydroxyl group, a carboxyl group, or the like, and a group for controlling the intermolecular cohesive force, for example, a long-chain hydrocarbon group, linked in the molecule.

### [Examples]

Hereinafter, the present invention will be described in detail with reference to Examples, but the present invention is not limited to the following Examples.

### (Example 1) Production of Compound Example 1

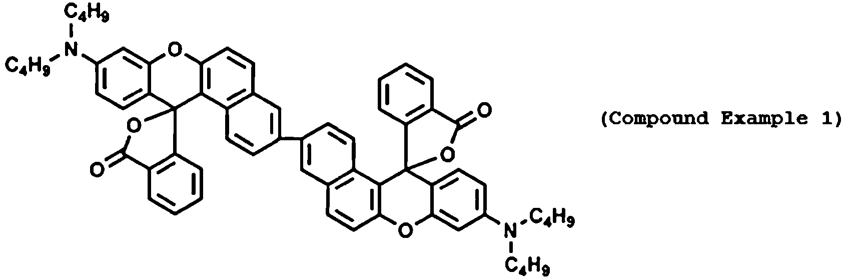

18.5 g of the compound (3-a) and 7.9 g of the compound (4-a) were mixed in 75 g of 98% sulfuric acid, and the mixture was stirred at 40°C to 50°C for 20 hours.

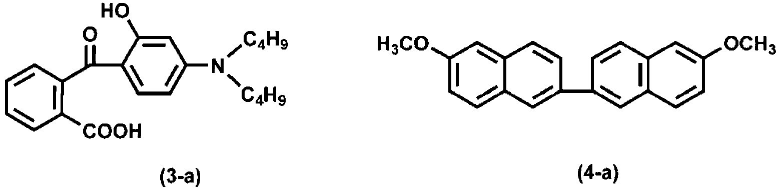

After cooling to room temperature, the reaction mixture was discharged into 400 g of ice water, and the precipitate was filtered off. The obtained precipitate was stirred with 200 mL of toluene and 100 g of a 25% sodium hydroxide aqueous solution under reflux for 1 hour, and after cooling, the toluene layer was separated and washed with hot water until neutral.

The toluene layer was concentrated under reduced pressure, 150 mL of methanol was added thereto, and the mixture was stirred under reflux for 1 hour. After cooling, the precipitate was collected by filtration, washed with 50 mL of methanol, and dried to obtain 18.7 g of a light pinkish white powder as a target substance.

It was confirmed that the obtained compound was a target compound based on the following analysis results.
ESI-Mass: 953 (M+H)+
Element analysis values: actual values (C: 80.62%, H: 6.30%, N: 2.98%)
Theoretical values (C: 80.65%, H: 6.34%, N: 2.94%)
5% weight loss temperature: 359.8°C

The 5% weight loss temperature is a temperature at which the weight is reduced by 5% by thermogravimetric-differential thermal analysis, and the same applies hereinafter.

### (Example 2) Production of Compound Example 2

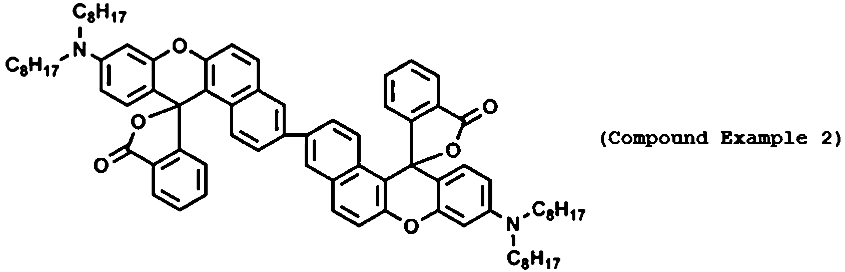

19.3 g of a white powder as a target substance was obtained by the same production method as in Example 1, except that 24.1 g of the compound (3-b) was used instead of 18.5 g of the compound (3-a) of Example 1.

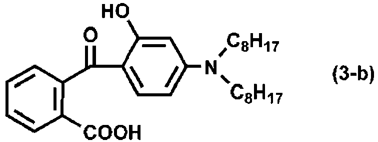

It was confirmed that the obtained compound was a target compound based on the following analysis results.
ESI-Mass: 1178 (M+H)+
Element analysis values: actual values (C: 81.57%, H: 7.83%, N: 2.41%)
Theoretical values (C: 81.59%, H: 7.87%, N: 2.38%)
5% weight loss temperature: 359.8°C

### (Example 3) Production of Compound Example 4

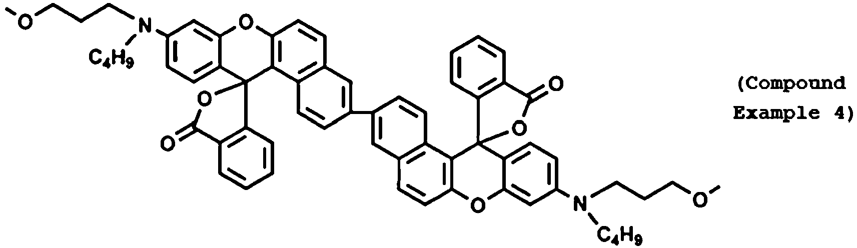

16.8 g of a light brownish white powder as a target substance was obtained by the same production method as in Example 1, except that 19.3 g of the compound (3-c) was used instead of 18.5 g of the compound (3-a) of Example 1.

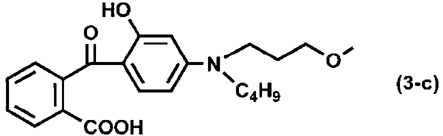

It was confirmed that the obtained compound was a target compound based on the following analysis results.
ESI-Mass: 985 (M+H)+
Element analysis values: actual values (C: 77.98%, H: 6.11%, N: 2.87%)
Theoretical values (C: 78.03%, H: 6.14%, N: 2.84%)
5% weight loss temperature: 353.4°C

### (Example 4) Production of Compound Example 9

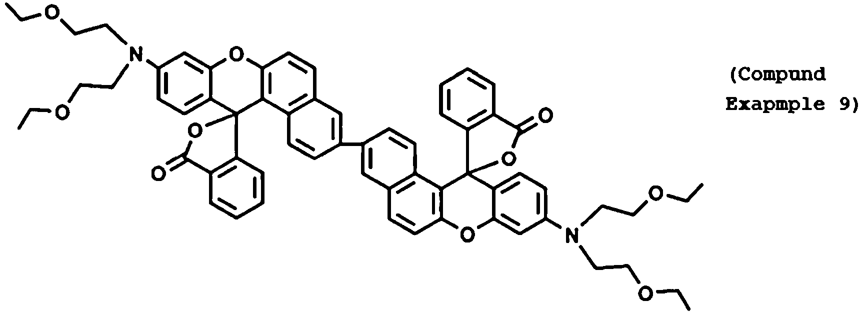

19.9 g of a light pinkish white powder, as a target substance, was obtained by the same production method as in Example 1, except that 20.1 g of the compound (3-d) was used instead of 18.5 g of the compound (3-a) of Example 1.

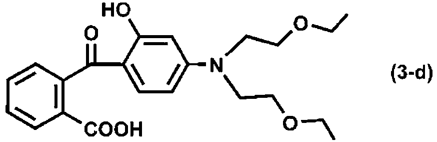

It was confirmed that the obtained compound was a target compound based on the following analysis results.
ESI-Mass: 1017 (M+H)+
Element analysis values: actual values (C: 75.53%, H: 5.90%, N: 2.79%)
Theoretical values (C: 75.57%, H: 5.95%, N: 2.75%)
5% weight loss temperature: 344.9°C

### (Example 5) Production of Compound Example 13

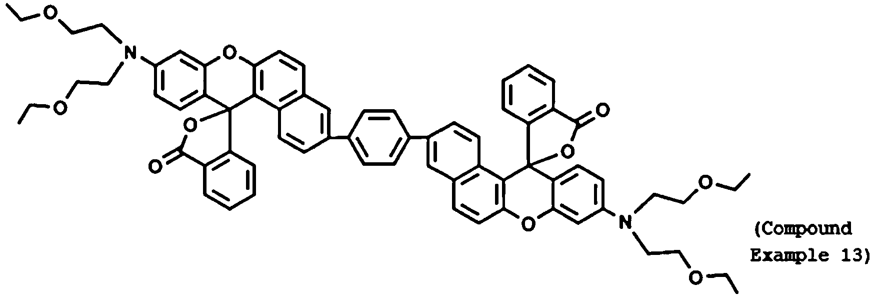

17.3 g of a light orange-white powder as a target substance was obtained by the same production method as in Example 1, except that 20.1 g of the compound (3-d) was used instead of 18.5 g of the compound (3-a) of Example 1 and 9.8 g of the compound (4-b) was used instead of 7.9 g of the compound (4-a).

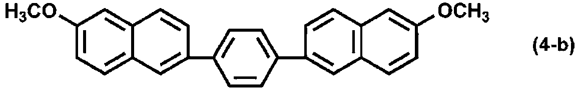

It was confirmed that the obtained compound was a target compound based on the following analysis results.
ESI-Mass: 1093 (M+H)+
Element analysis values: actual values (C: 76.86%, H: 5.88%, N: 2.58%)
Theoretical values (C: 76.90%, H: 5.90%, N: 2.56%)
5% weight loss temperature: 344.1°C

### (Example 6) Production of Compound Example 23

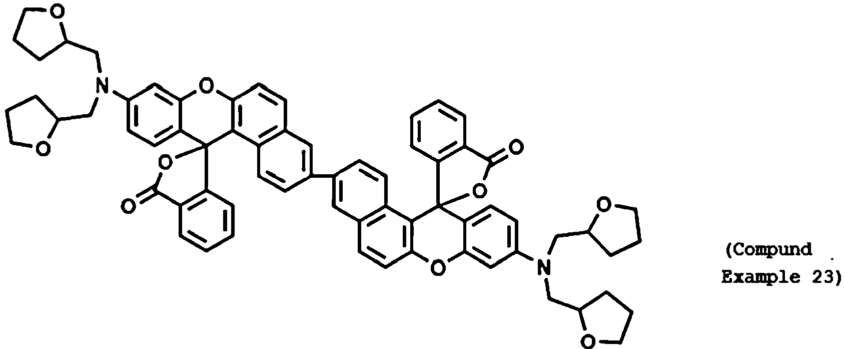

15.3 g of a pale violet-white powder as a target substance was obtained by the same production method as in Example 1, except that 21.3 g of the compound (3-e) was used instead of 18.5 g of the compound (3-a) of Example 1.

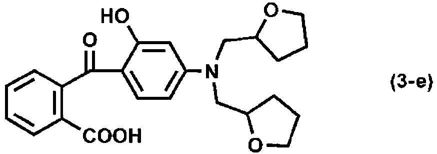

It was confirmed that the obtained compound was a target compound based on the following analysis results.
ESI-Mass: 1065 (M+H)+
Element analysis values: actual values (C: 76.65%, H: 5.64%, N: 2.66%)
Theoretical values (C: 76.67%, H: 5.68%, N: 2.63%)
5% weight loss temperature: 355.0°C

### (Example 7) Production of Compound Example 35

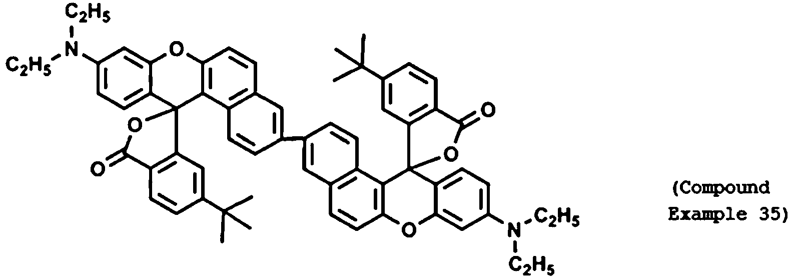

20.8 g of a pale violet-white powder as a target substance was obtained by the same production method as in Example 1, except that 18.5 g of the compound (3-f) was used instead of 18.5 g of the compound (3-a) of Example 1.

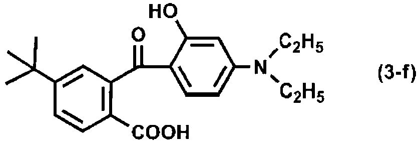

It was confirmed that the obtained compound was a target compound based on the following analysis results.
ESI-Mass: 953 (M+H)+
Element analysis values: actual values (C: 80.61%, H: 6.29%, N: 2.97%)
Theoretical values (C: 80.65%, H: 6.34%, N: 2.94%)
5% weight loss temperature: 361.5°C

### (Example 8) Production of Compound Example 36

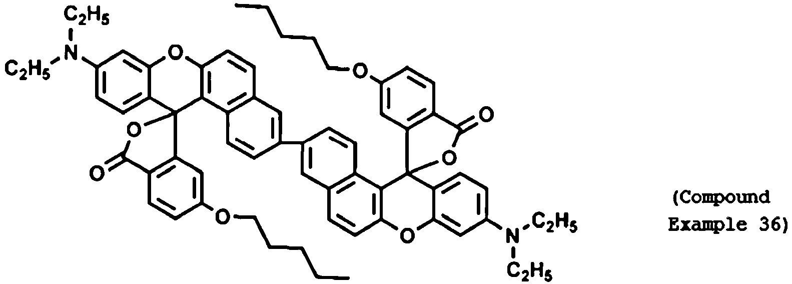

17.1 g of a pale violet-white powder as a target substance was obtained by the same production method as in Example 1, except that 20.0 g of the compound (3-g) was used instead of 18.5 g of the compound (3-a) of Example 1.

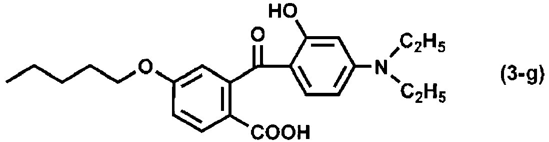

It was confirmed that the obtained compound was a target compound based on the following analysis results.
ESI-Mass: 1013 (M+H)+
Element analysis values: actual values (C: 78.20%, H: 6.33%, N: 2.79%)
Theoretical values (C: 78.24%, H: 6.37%, N: 2.76%)
5% weight loss temperature: 310.4°C

### (Example 9) Production of Compound Example 47

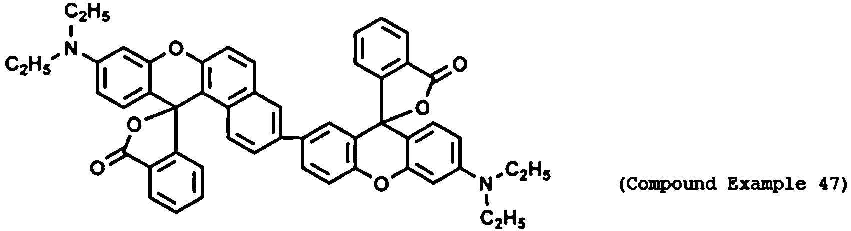

16.0 g of a light pinkish white powder as a target substance was obtained by the same production method as in Example 1, except that 15.7 g of the compound (3-h) was used instead of 18.5 g of the compound (3-a) of Example 1 and 6.6 g of the compound (4-c) was used instead of 7.9 g of the compound (4-a).

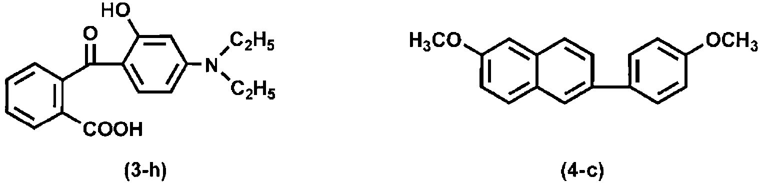

It was confirmed that the obtained compound was a target compound based on the following analysis results.
ESI-Mass: 791 (M+H)+
Element analysis values: actual values (C: 78.93%, H: 5.31%, N: 3.60%)
Theoretical values (C: 78.97%, H: 5.35%, N: 3.54%)
5% weight loss temperature: 165.3°C

### (Example 10) Production of Compound Example 48

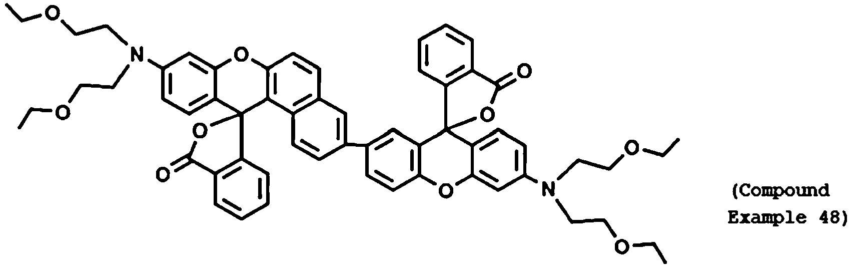

14.0 g of a white powder was obtained by the same production method as in Example 1, except that 20.1 g of the compound (3-d) was used instead of 18.5 g of the compound (3-a) of Example 1 and 6.6 g of the compound (4-c) was used instead of 7.9 g of the compound (4-a).

It was confirmed that the obtained compound was a target compound based on the following analysis results.
ESI-Mass: 967 (M+H)+
Element analysis values: actual values (C: 74.48%, H: 6.02%, N: 2.92%)
Theoretical values (C: 74.52%, H: 6.05%, N: 2.90%)
5% weight loss temperature: 345.4°C

### (Example 11) Production of Compound Example 71

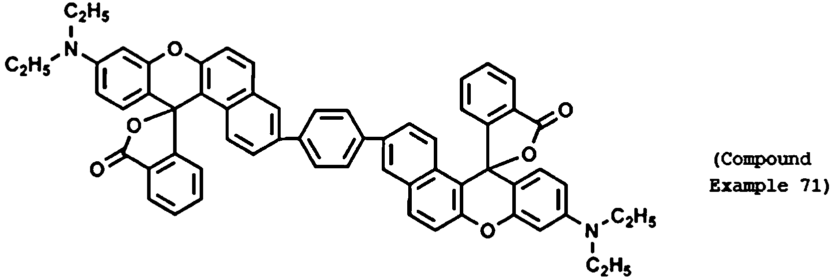

14.5 g of a light pinkish white powder was obtained by the same production method as in Example 1, except that 15.7 g of the compound (3-h) was used instead of 18.5 g of the compound (3-a) of Example 1 and 9.8 g of the compound (4-b) was used instead of 7.9 g of the compound (4-a) of Example 1.

It was confirmed that the obtained compound was a target compound based on the following analysis results.
ESI-Mass: 917 (M+H)+
Element analysis values: actual values (C: 81.17%, H: 5.26%, N: 3.08%)
Theoretical values (C: 81.20%, H: 5.28%, N: 3.05%)
5% weight loss temperature: 339.7°C

### (Example 12) Production of Compound Example 73

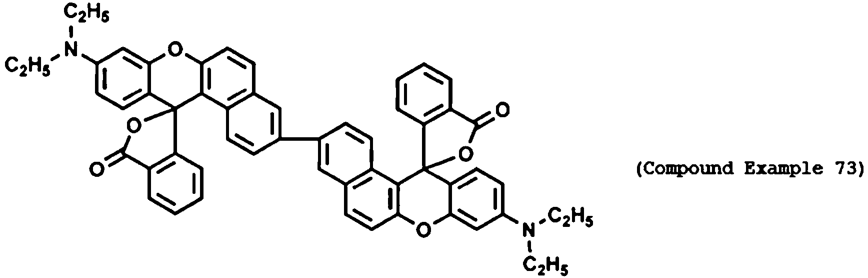

15.3 g of a light brownish white powder was obtained by the same production method as in Example 1, except that 15.7 g of the compound (3-h) was used instead of 18.5 g of the compound (3-a) of Example 1.

It was confirmed that the obtained compound was a target compound based on the following analysis results.
ESI-Mass: 841 (M+H)+
Element analysis values: actual values (C: 79.95%, H: 5.26%, N: 3.36%)
Theoretical values (C: 79.98%, H: 5.27%, N: 3.33%)
5% weight loss temperature: 356.3°C

### (Example 13) Production of Compound Example 75

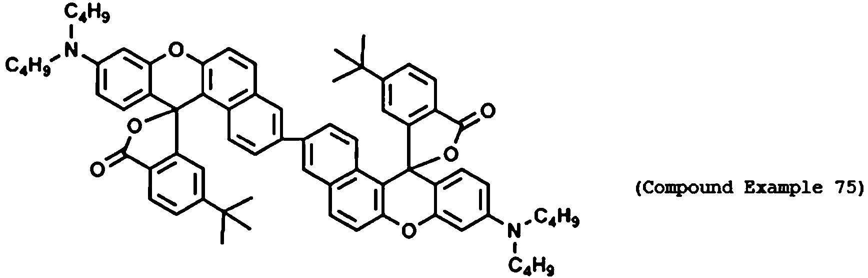

24.3 g of a light orange-white powder was obtained by the same production method as in Example 1, except that 21.3 g of the compound (3-i) was used instead of 18.5 g of the compound (3-a) of Example 1.

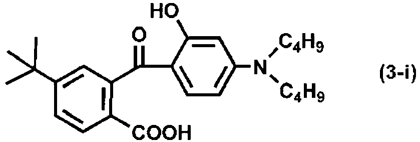

It was confirmed that the obtained compound was a target compound based on the following analysis results.
ESI-Mass: 1065 (M+H)+
Element analysis values: actual values (C: 81.13%, H: 7.15%, N: 2.67%)
Theoretical values (C: 81.17%, H: 7.19%, N: 2.63%)
5% weight loss temperature: 354.5°C

### (Example 14) Production of Compound Example 79

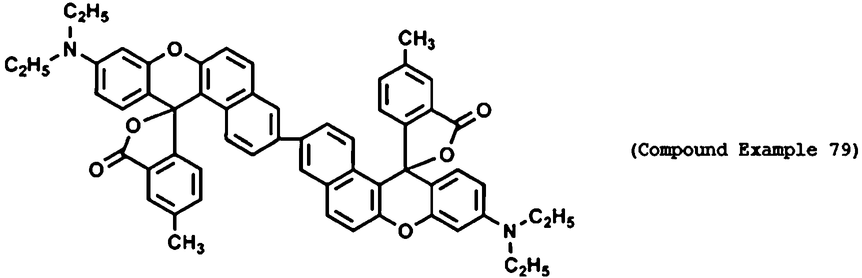

18.3 g of light pinkish white powder was obtained by the same production method as in Example 1, except that 16.4 g of the compound (3-j) was used instead of 18.5 g of the compound (3-a) of Example 1.

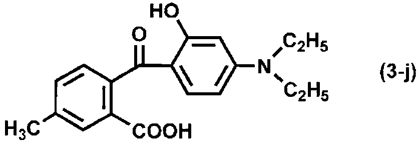

It was confirmed that the obtained compound was a target compound based on the following analysis results.
ESI-Mass: 869 (M+H)+
Element analysis values: actual values (C: 80.13%, H: 5.53%, N: 3.25%)
Theoretical values (C: 80.16%, H: 5.57%, N: 3.22%)
5% weight loss temperature: 370.5°C

### (Example 15) Production of Compound Example 80

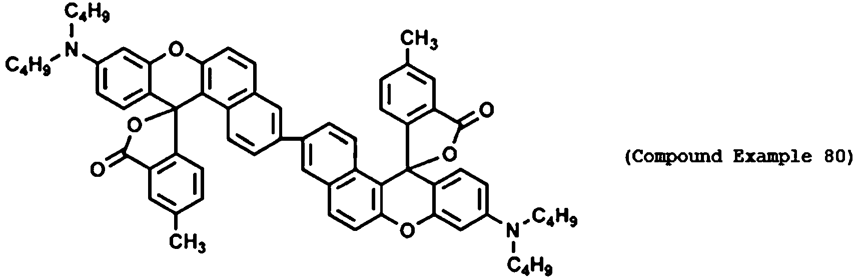

19.0 g of a light brownish white powder was obtained by the same production method as in Example 1, except that 19.2 g of the compound (3-k) was used instead of 18.5 g of the compound (3-a) of Example 1.

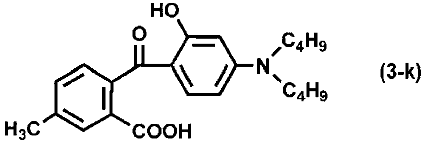

It was confirmed that the obtained compound was a target compound based on the following analysis results.
ESI-Mass: 981 (M+H)+
Element analysis values: actual value (C: 80.77%, H: 6.53%, N: 2.87%)
Theoretical values (C: 80.79%, H: 6.57%, N: 2.85%)
5% weight loss temperature: 357.2°C

### (Example 16) Production of Compound Example 91

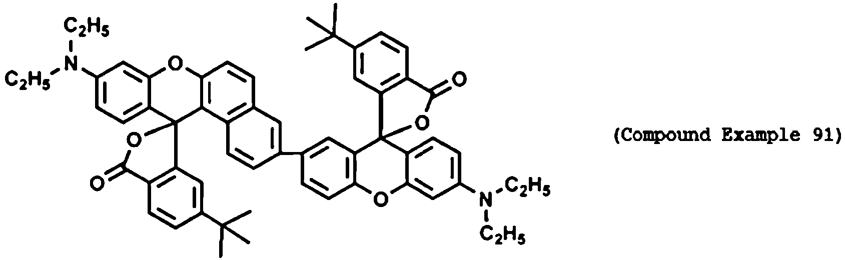

17.0 g of a light reddish beige powder was obtained by the same production method as in Example 1, except that 18.5 g of the compound (3-f) was used instead of 18.5 g of the compound (3-a) of Example 1, and 6.6 g of the compound (4-c) was used instead of 7.9 g of the compound (4-a).

It was confirmed that the obtained compound was a target compound based on the following analysis results.
ESI-Mass: 903 (M+H)+
Element analysis values: actual value (C: 79.78%, H: 6.44%, N: 3.13%)
Theoretical values (C: 79.80%, H: 6.47%, N: 3.10%)
5% weight loss temperature: 356.0°C

### (Example 17) Production of Compound Example 92

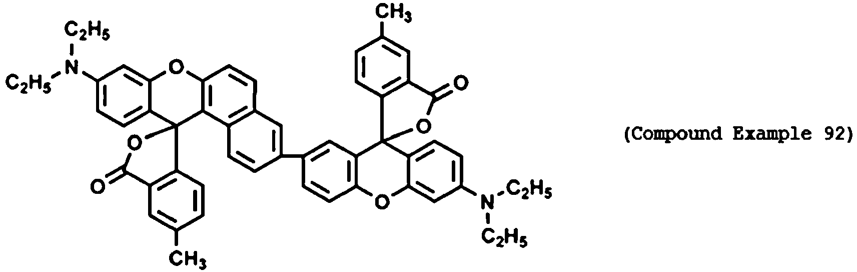

18.0 g of a light reddish beige powder was obtained by the same production method as in Example 1, except that 16.4 g of the compound (3-j) was used instead of 18.5 g of the compound (3-a) of Example 1, and 6.6 g of the compound (4-c) was used instead of 7.9 g of the compound (4-a).

It was confirmed that the obtained compound was a target compound based on the following analysis results.
ESI-Mass: 819 (M+H)+
Element analysis values: actual value (C: 79.18%, H: 5.63%, N: 3.45%)
Theoretical values (C: 79.20%, H: 5.66%, N: 3.42%)
5% weight loss temperature: 355.3°C

### (Example 18) Production of Compound Example 93

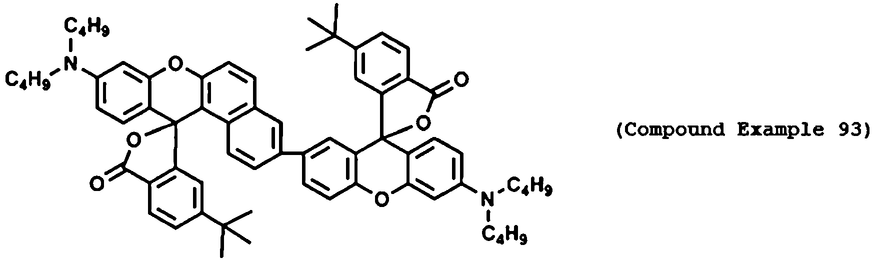

21.1 g of a beige powder was obtained by the same production method as in Example 1, except that 21.3 g of the compound (3-i) was used instead of 18.5 g of the compound (3-a) of Example 1, and 6.6 g of the compound (4-c) was used instead of 7.9 g of the compound (4-a).

It was confirmed that the obtained compound was a target compound based on the following analysis results.
ESI-Mass: 1015 (M+H)+
Element analysis values: actual value (C: 80.41%, H: 7.33%, N: 2.79%)
Theoretical values (C: 80.44%, H: 7.35%, N: 2.76%)
5% weight loss temperature: 353.8°C

### (Example 19) Production of Compound Example 94

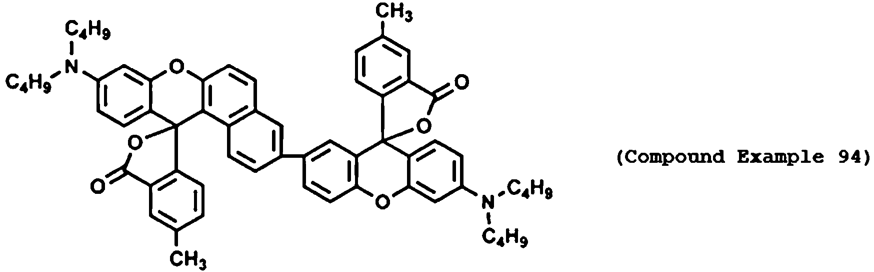

16.5 g of a light brown powder was obtained by the same production method as in Example 1, except that 19.2 g of the compound (3-k) was used instead of 18.5 g of the compound (3-a) of Example 1, and 6.6 g of the compound (4-c) was used instead of 7.9 g of the compound (4-a).

It was confirmed that the obtained compound was a target compound based on the following analysis results.
ESI-Mass: 931 (M+H)+
Element analysis values: actual value (C: 79.95%, H: 6.69%, N: 3.05%)
Theoretical values (C: 79.97%, H: 6.71%, N: 3.01%)
5% weight loss temperature: 343.5°C

### (Example 20) Production of Compound Example 123

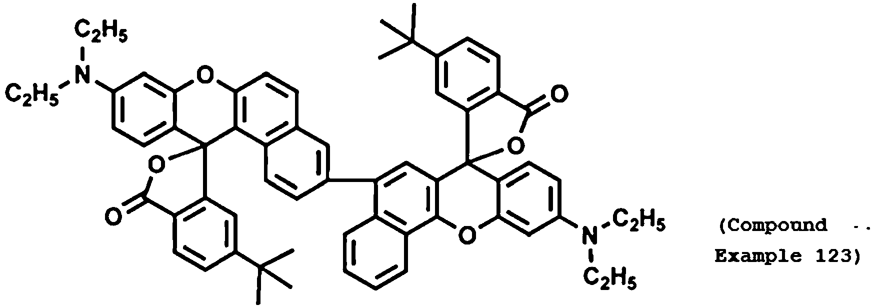

19.8 g of a light brownish white powder as a target substance was obtained by the same production method as in Example 7, except that 7.9 g of the compound (4-d) was used instead of 7.9 g of the compound (4-a) in Example 7.

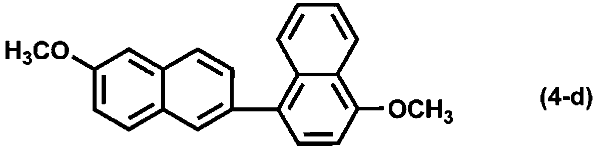

It was confirmed that the obtained compound was a target compound based on the following analysis results.
ESI-Mass: 953 (M+H)+
Element analysis values: actual values (C: 80.59%, H: 6.31%, N: 2.97%)
Theoretical values (C: 80.65%, H: 6.34%, N: 2.94%)
5% weight loss temperature: 346.2°C

### (Example 21) Production of Compound Example 124

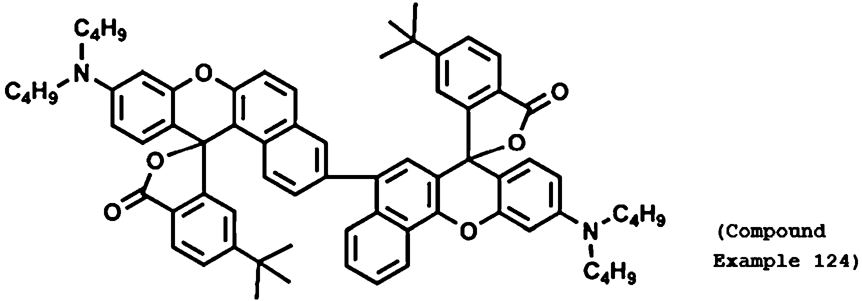

15.8 g of a light brownish white powder as a target substance was obtained by the same production method as in Example 13, except that 7.9 g of the compound (4-d) was used instead of 7.9 g of the compound (4-a) of Example 13.

It was confirmed that the obtained compound was a target compound based on the following analysis results.
ESI-Mass: 1065 (M+H)+
Element analysis values: actual values (C: 81.15%, H: 7.23%, N: 2.60%)
Theoretical values (C: 81.17%, H: 7.19%, N: 2.63%)
5% weight loss temperature: 341.2°C

### (Example 22) Production of Compound Example 125

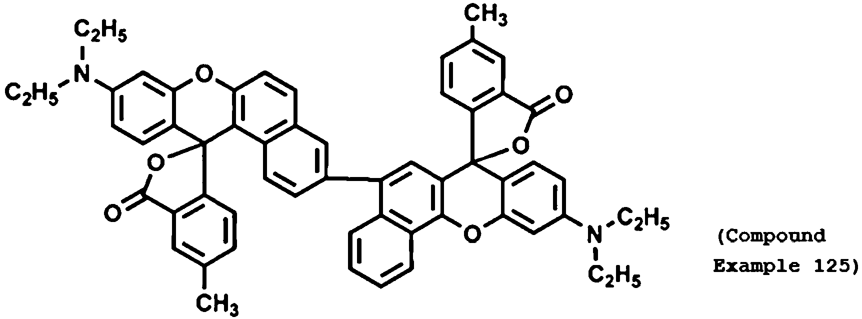

13.9 g of a light brownish white powder as a target substance was obtained by the same production method as in Example 14, except that 7.9 g of the compound (4-d) was used instead of 7.9 g of the compound (4-a) of Example 14.

It was confirmed that the obtained compound was a target compound based on the following analysis results.
ESI-Mass: 869 (M+H)+
Element analysis values: actual values (C: 80.13%, H: 5.60%, N: 3.18%)
Theoretical values (C: 80.16%, H: 5.57%, N: 3.22%)
5% weight loss temperature: 349.2°C

### (Example 23) Production of Compound Example 126

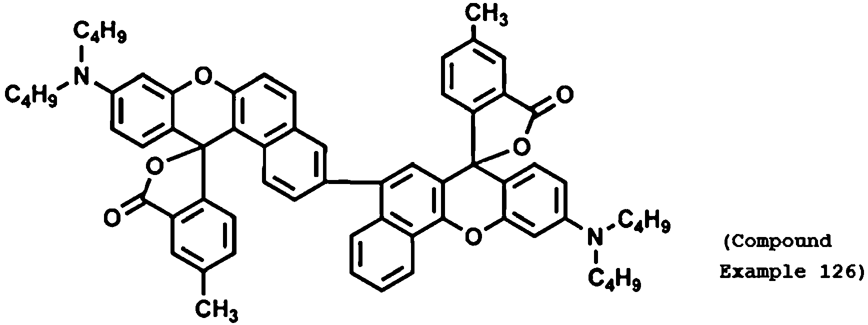

15.2 g of a light brownish white powder as a target substance was obtained by the same production method as in Example 15, except that 7.9 g of the compound (4-d) was used instead of 7.9 g of the compound (4-a) of Example 15.

It was confirmed that the obtained compound was a target compound based on the following analysis results.
ESI-Mass: 981 (M+H)+
Element analysis values: actual values (C: 80.75%, H: 6.60%, N: 2.81%)
Theoretical values (C: 80.79%, H: 6.57%, N: 2.85%)
5% weight loss temperature: 341.5°C

### (Examples 24 to 46) Preparation of Test Thermosensitive Recording Material

For Examples 1 to 23, a test thermosensitive recording material was prepared, and the following evaluations were performed. In addition, as a comparative example, the same test thermosensitive recording material was prepared using the following comparative compound 1, and the evaluation was performed.

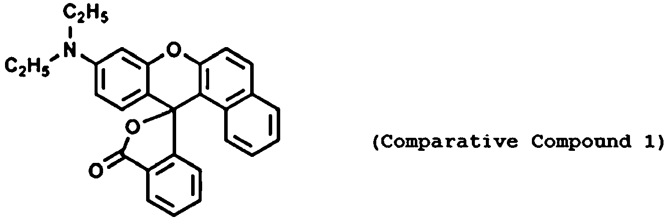

### [Preparation of Test Thermosensitive Recording Material]

45 g of a 2.5% polyvinyl alcohol aqueous solution and 5 g of each of the compounds produced in Examples 1 to 23 and the comparative compound were mixed, and the mixture was pulverized so that the average particle diameter was 1 micron, thereby producing a dispersion liquid.

On the other hand, 10 g of bisphenol A and 10 g of parabenzylbiphenyl were pulverized together with 80 g of a 2.5% polyvinyl alcohol aqueous solution using a sand mill so that the average particle diameter was 3 µm, thereby producing a dispersion liquid.

After mixing the two types of dispersion liquids produced in this manner, 30 g of a 50% calcium carbonate dispersion liquid and a 30% paraffin wax dispersion liquid were added thereto and mixed well, thereby preparing a thermosensitive coating liquid.

The thermosensitive coating liquid prepared in this manner was applied to high-quality paper having a basis weight of 50 g/m² so that the solid content application amount was 5 g/m², and after drying, a calender treatment was performed so that the Bekk smoothness of the thermosensitive recording surface was 400 to 500 seconds, thereby producing a white test thermosensitive recording material.

### [Quality Performance Test]

Each of the test thermosensitive recording materials according to Examples and Comparative Examples was evaluated as follows.

### (Measurement of Base Whiteness)

Using a reflection densitometer RD-914 manufactured by Macbeth, the whiteness of the uncolored portion of the test thermosensitive recording material was measured by an OD value. The results are listed in Table 1. The lower the numerical value, the higher the whiteness of the uncolored portion.

### (Measurement of Base Stability)

The test thermosensitive recording material was irradiated with a fluorescent lamp of 20,000 lux for 72 hours, and then the light fastness of the base was measured by the base coloration density (OD value) with a reflection densitometer RD-914.

Similarly, the test thermosensitive recording material was kept warm in an electric dryer DS44 manufactured by Yamato Scientific Co., Ltd. at 60°C for 24 hours, and then the heat resistance of the base was measured by the base coloration density (OD value) with a reflection densitometer RD-914.

The results are listed in Table 1. The lower the numerical value after the test, the higher the stability of the base.

### (Stability Test of Developed Color Image)

Using a printing device TH-PMD manufactured by Okura Electric Co., Ltd., the test thermosensitive recording material was printed by adjusting the pulse width such that the color image density (OD value) was about 1.1, thereby obtaining a developed color image of magenta color.

The following tests were performed on the obtained developed color image. The density of the residual image after each test was measured by an OD value with a reflection densitometer RD-914 manufactured by Macbeth, and the stability of the developed color image was evaluated.

For the light fastness, the developed color image was irradiated with a fluorescent lamp of 20,000 lux at 25°C for 72 hours, and then the residual image density was measured.

For the moisture-heat resistance, the developed color image was kept in a constant temperature and humidity chamber LHU-112M manufactured by TABI-ESPEC CORP. at 90% RH and 50°C for 24 hours, and then the residual image density was measured.

For the heat resistance, the developed color image was kept warm in an electric dryer DS44 manufactured by Yamato Scientific Co., Ltd. at 60°C for 24 hours, and then the residual image density was measured.

Each stability of the developed color image is represented by the following expression. 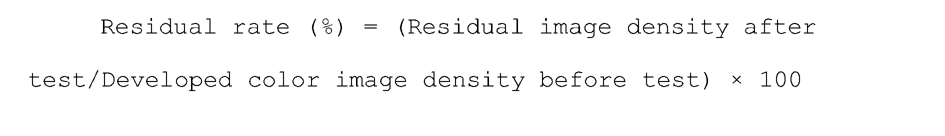 Residual rate (%) = (Residual image density after test/Developed color image density before test) × 100

The results are listed in Table 1. The higher the numerical value of the residual rate, the higher each stability of the developed color image.

**Table 1**

| | Compound | Base whiteness (OD value) | Base stability | | Image fastness | | |
|---|---|---|---|---|---|---|---|
| | | | Heat resistance (OD value) | Light fastness (OD value) | Moisture-heat resistance (%) | Heat resistance (%) | Light fastness (%) |
| Example 24 | Compound Example 1 | 0.09 | 0.13 | 0.16 | 92 | 86 | 91 |
| Example 25 | Compound Example 2 | 0.09 | 0.12 | 0.15 | 92 | 87 | 90 |
| Example 26 | Compound Example 4 | 0.10 | 0.15 | 0.17 | 91 | 86 | 91 |
| Example 27 | Compound Example 9 | 0.11 | 0.14 | 0.18 | 90 | 85 | 89 |
| Example 28 | Compound Example 13 | 0.10 | 0.15 | 0.16 | 91 | 86 | 90 |
| Example 29 | Compound Example 23 | 0.11 | 0.15 | 0.16 | 89 | 84 | 88 |
| Example 30 | Compound Example 35 | 0.08 | 0.11 | 0.12 | 94 | 89 | 93 |
| Example 31 | Compound Example 36 | 0.10 | 0.14 | 0.17 | 92 | 86 | 91 |
| Example 32 | Compound Example 47 | 0.09 | 0.13 | 0.15 | 92 | 87 | 91 |
| Example 33 | Compound Example 48 | 0.10 | 0.16 | 0.17 | 90 | 85 | 90 |
| Example 34 | Compound Example 71 | 0.09 | 0.13 | 0.14 | 92 | 88 | 91 |
| Example 35 | Compound Example 73 | 0.09 | 0.12 | 0.15 | 92 | 88 | 92 |
| Example 36 | Compound Example 75 | 0.08 | 0.12 | 0.13 | 93 | 88 | 93 |
| Example 37 | Compound Example 79 | 0.08 | 0.13 | 0.14 | 92 | 87 | 92 |
| Example 38 | Compound Example 80 | 0.08 | 0.14 | 0.13 | 92 | 88 | 92 |
| Example 39 | Compound Example 91 | 0.08 | 0.12 | 0.12 | 92 | 88 | 93 |
| Example 40 | Compound Example 92 | 0.08 | 0.12 | 0.14 | 91 | 87 | 91 |
| Example 41 | Compound Example 93 | 0.09 | 0.13 | 0.15 | 90 | 85 | 90 |
| Example 42 | Compound Example 94 | 0.10 | 0.15 | 0.17 | 91 | 86 | 91 |
| Example 43 | Compound Example 123 | 0.08 | 0.1 | 0.12 | 93 | 88 | 92 |
| Example 44 | Compound Example 124 | 0.08 | 0.1 | 0.13 | 91 | 86 | 90 |
| Example 45 | Compound Example 125 | 0.10 | 0.11 | 0.14 | 89 | 87 | 91 |
| Example 46 | Compound Example 126 | 0.09 | 0.11 | 0.12 | 90 | 87 | 90 |
| Comparative Example 1 | Comparative Compound 1 | 0.12 | 0.18 | 0.31 | 77 | 76 | 83 |

### [Industrial Applicability]

The recording material using the fluoran dimer compound according to the present invention has excellent whiteness of the base and excellent heat resistance and light fastness in the base stability before color development, and thus has excellent storage stability. Further, after color development, all the performances of moisture-heat resistance, heat resistance, and light fastness in the image fastness are excellent, and thus a clear magenta developed color image can be stably provided.

As described above, the fluoran dimer compound according to the present invention is very useful as a color forming agent of a thermosensitive recording material.

This application claims priority based on Japanese Patent Application No. 2023-184457 filed on October 27, 2023, the entirety of the disclosure of which is incorporated herein.

## Claims

1. A fluoran dimer compound represented by General Formula (1), 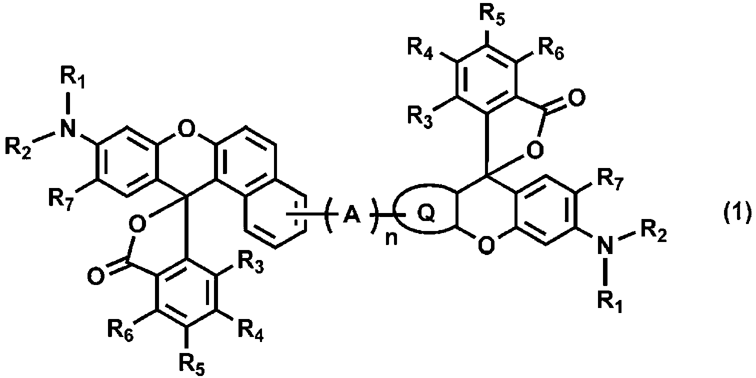
wherein, in Formula (1), R₁ and R₂ each independently represent a hydrogen atom, a substituted or unsubstituted alkyl group, a substituted or unsubstituted aralkyl group, or a substituted or unsubstituted aryl group, and R₁ and R₂ are optionally bonded to each other to form an aliphatic ring; R₃ to R₆ each independently represent a hydrogen atom, a halogen atom, a substituted or unsubstituted alkyl group, a substituted or unsubstituted alkoxy group, a substituted or unsubstituted aryloxy group, a substituted or unsubstituted alkylthio group, or a substituted or unsubstituted arylthio group, and R₃ to R₆ are optionally bonded to each other to form an aliphatic ring or an aromatic ring;
R₇ represents a hydrogen atom or an unsubstituted alkyl group;
Q represents an unsubstituted benzene ring or an unsubstituted naphthalene ring; A is selected from a substituted or unsubstituted arylene group or a group represented by General Formula (2); and n represents 0 or 1, 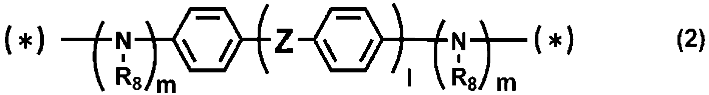
wherein, in Formula (2), Z represents an oxygen atom, a sulfur atom, a phenylene group, a ketone group, or a substituted or unsubstituted alkylene group, R₈ represents a hydrogen atom or an unsubstituted alkyl group; and l and m each independently represent 0 or 1, in a case where l is 0, m represents 1, and in a case where m is 0, l represents 1; and (*) represents a bonding position.

2. The fluoran dimer compound according to Claim 1, wherein Q is an unsubstituted benzene ring.

3. The fluoran dimer compound according to Claim 1, wherein Q is an unsubstituted naphthalene ring.

4. The fluoran dimer compound according to Claim 1, wherein R₁ and R₂ each independently represent a hydrogen atom, a substituted or unsubstituted alkyl group having 1 to 20 carbon atoms, a substituted or unsubstituted aralkyl group having 7 to 25 carbon atoms, or a substituted or unsubstituted aryl group having 6 to 24 carbon atoms, and R₁ and R₂ are optionally bonded to each other to form a substituted or unsubstituted heterocyclic aliphatic ring having 4 to 20 carbon atoms.

5. The fluoran dimer compound according to Claim 1, wherein R₃ to R₆ each independently represent a hydrogen atom, a halogen atom, a substituted or unsubstituted alkyl group having 1 to 20 carbon atoms, a substituted or unsubstituted alkoxy group having 1 to 25 carbon atoms, a substituted or unsubstituted aryloxy group having 6 to 25 carbon atoms, a substituted or unsubstituted alkylthio group having 1 to 25 carbon atoms, or a substituted or unsubstituted arylthio group having 6 to 25 carbon atoms, and R₃ to R₆ are optionally bonded to each other to form a substituted or unsubstituted carbocyclic aliphatic ring having 5 to 20 carbon atoms or a substituted or unsubstituted carbocyclic aromatic ring having 6 to 20 carbon atoms.

6. The fluoran dimer compound according to Claim 1, wherein R₇ represents a hydrogen atom or an unsubstituted alkyl group having 1 to 8 carbon atoms.

7. The fluoran dimer compound according to Claim 1,
wherein A is a substituted or unsubstituted arylene group having 6 to 18 carbon atoms or a group represented by General Formula (2),
wherein, in General Formula (2), Z represents an oxygen atom, a sulfur atom, a ketone group, or a substituted or unsubstituted alkylene group having 1 to 20 carbon atoms, and R₈ represents a hydrogen atom or an alkyl group having 1 to 4 carbon atoms.

8. A recording material comprising:
an electron-donating color forming agent; and an electron-accepting color developer, wherein a color-forming reaction between the electron-donating color forming agent and the electron-accepting color developer is utilized, and the electron-donating color forming agent includes at least one of the fluoran dimer compounds according to any one of Claims 1 to 7.

9. A thermosensitive recording material comprising the recording material according to Claim 8.
